# EUROPEAN PATENT SPECIFICATION

(11) **EP 2 185 078 B1**
(45) Date of publication and mention of the grant of the patent: **03.12.2014**
(21) Application number: 08828617.4
(22) Date of filing: 01.08.2008
(51) Int. Cl.: A61B 8/12, A61B 8/00

(54) **WIRELESS REMOTELY CONTROLLED ULTRASONIC TRANSDUCER AND IMAGING APPARATUS**
KABELLOSER FERNGESTEUERTER ULTRASCHALLWANDLER UND ABBILDUNGSVORRICHTUNG
TRANSDUCTEUR À ULTRASONS COMMANDÉ À DISTANCE, SANS FIL, ET APPAREIL D'IMAGERIE

(30) Priority: 03.08.2007 US 953861 P; 30.07.2008 US 182247
(43) Date of publication of application: 19.05.2010
(73) Proprietor: Innoscion LLC, Baltimore MD 21210 (US)
(72) Inventor: ABRAHAM, Theodore, P., Baltimore, MD 21210 (US)
(74) Representative: Mitchell, Matthew Benedict David
(86) International application number: PCT/US2008/071943
(87) International publication number: WO 2009/029388

(56) References cited:
- EP-A1- 0 607 490
- EP-A2- 1 286 172
- EP-A2- 1 566 201
- WO-A1-01/12068
- WO-A1-2006/036870
- WO-A1-2006/131881
- WO-A2-02/094089
- WO-A2-2007/014292
- DE-A1- 3 719 919
- KR-A- 20050 001 074
- US-A- 3 893 449
- US-A- 4 341 120
- US-A- 4 442 844
- US-A- 4 458 689
- US-A- 5 195 519
- US-A- 5 360 005
- US-A- 5 409 005
- US-A1- 2004 171 935
- US-B1- 6 406 431

## Description

### TECHNICAL FIELD

The technical field of the several embodiments of a wired or wireless ultrasonic transducer relates generally to the field of ultrasound and related medical imaging methods and apparatus and, more particularly, to embodiments thereof, for example, wherein the ultrasonic arrays and imaging apparatus may include motors and the assembly affixed to a patient and the transducers remotely controlled as to rotation, linear movement, pitch and, generally, direction and depth of imaging, in addition to other control aspects and features, such that imaging and control thereof may be conducted remotely from the patient with minimal invasion of patient privacy.

### BACKGROUND

Ultrasonic imaging is known in the art and systems typically involve the use of hand-held or temporarily affixed ultrasonic transducer element or transducer arrays that may be controlled, for example, as to on/off, mode, focus control, depth control and the like. A qualified user applies a small amount of ultrasound gel to a region of interest, holds and moves the ultrasound transducer from one location of the patient to another within the region, the unit being wired to a console, typically including a display. The ultrasound transducer is housed in a wand that may be moved about the patient's body surface to obtain two dimensional, three dimensional or so- called four dimensional (including movement over time of the subject of the imaging) views provided on a display of the console of the region of interest. It is generally known that one may improve the image by moving the wand slightly, for example, to avoid reflections from internal body parts that are not of interest and thus obtain an improved image of a body part, such as the heart, that is of greater interest and to obtain a stronger reflected signal from the heart. Ultrasound is a biologically safe and non-radiating form of energy that can provide detailed anatomic and, in some cases, functional images. It is known in the art of transesophageal echocardiography (imagery of the heart) to provide a multi-plane transducer that can image in planes in a 180 degree range.

The advent of piezocomposite material comprising a piezoelectric ceramic and a polymer has improved performance of commonly used ultrasonic arrays. Ultrasonic arrays may be annular, rectangular (linear) and two dimensional. While originally analog, ultrasound imaging is now digital in order from transducer to beam former, signal processor, scan converter and a monitor or display. Imaging is similar to television reception as scan lines of an image are produced at a depth of field given by the equation 6 x (Lamda) x (FN)² where FN is the ratio of focal depth and aperture. A plurality of lines of ultrasound picture elements (pixels) comprise a frame and a typical frame rate is 30 frames per second. For example, a transducer operating at 10 MHz operating at an f-number of 15 yields a depth of focus of about 2 cm. In a phased-array system, elements of an array are used for each interrogation pulse and various time delays are introduced between the elements. Beam steering is accomplished by varying the time delays of the individual elements. A linear array is a stacking of adjacent elements linearly, for example, 512 elements over a 75 to 120 mm length. Subgroups of elements are pulsed in delay relation to other subgroups. Annular (circular) arrays vary in construction. An electronically steered-beam, phased-array has proven useful in cardiac imaging. On the other hand, phased-array transducers have not seen as extensive use as linear sequenced arrays in general ultrasound medical imaging. Real-time three dimensional imaging is known for providing motion imaging of a three dimensional space such as a pericardial cavity. Specialty arrays are known, for example, a curvilinear or convex abdominal transducer is known for providing an improved fit to an obstetric abdomen. The term "array" as used herein may be defined as a plurality of transducer elements in any one of a plurality of forms suitable to a particular purpose. Harmonic imaging is known for the transmission of a first frequency and the reception of reflections at a harmonic of the transmitted first frequency. The lower frequency transmission improves depth of penetration and the higher harmonic reception improves imaging particularly in imaging more obese patients.

Ultrasound operates on a principle of transmitting a sound wave of a given frequency range and recording the time and value of reflected wave data of a principal frequency and its harmonics from body parts of interest to the qualified user. Similarly, optical coherence tomography (OCT) utilizes a similar transmission/reflection process by transmitting light waves and recording the reflected light amplitude and delay. Light, however, may only penetrate a body to a depth of a few millimeters. Consequently, OCT has proven useful, for example, for quantifying the depth and healing over time of a third degree skin burn and underlying regions. Another field of medical imaging is infrared medical imaging where infrared energy is passively emitted by a body and provides an indication of temperature of the region imaged. An infrared camera captures the images of the infrared emission gradients which can be calibrated to show differences in temperature of adjacent regions, for example, as a yellow, orange, red scale. Infrared imaging has been found useful for imaging breast tumors and other close to the skin surface abnormalities indicated by temperature gradient.

It is also known, for example, in the telecommunications arts to remotely transmit images such as photographic images from a source such as a cellular telephone device to a receiving cellular telephone or other telecommunications device. For example, a doctor may transmit a digital image to another doctor by attaching the image to an email. An x-ray machine located in a remote laboratory may capture an image of a broken bone, and the technician may immediately transmit the image to an orthopedic unit of a hospital for analysis. Cellular telephone devices are now capable of capturing and transmitting moving images, including movies with associated sound, for personal enjoyment.

United States patent application Serial No. 11/782,991, filed July 25, 2007, entitled "Image Guided Catheters and Methods of Use," of the same inventor describes a plurality of embodiments of an image guided catheter that may be used, for example, to image an area of the thoracic cavity such as the heart or other region of interest and deliver medication, treatment and the like accompanied by ultrasonic and other imaging with an ultrasonic array mounted towards a distal end of a catheter. The catheter is provided with a plurality of lumen running from a proximal to the distal end. Interventional, diagnostic or therapeutic devices may be inserted via a sheath to the region of interest.

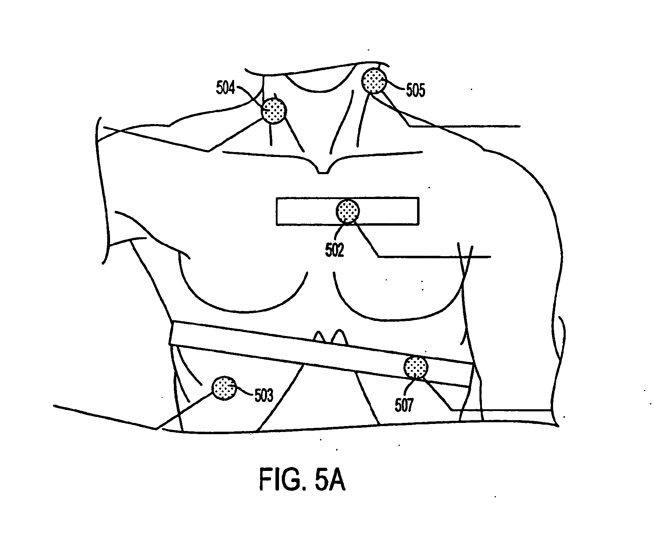

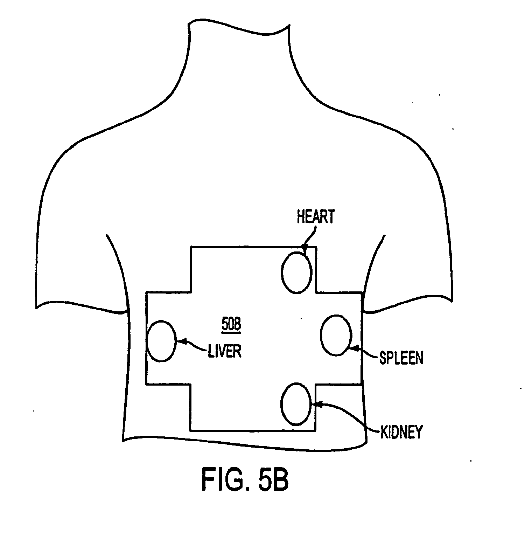

United States Patent No. 5,465,724 issued November 14, 1995 to Sliwa, Jr. et al. discloses a compact rotationally steerable ultrasound transducer having a circular track or a carrier band operable to rotate a multielement transducer, for example for transesophegeal echocardiography. United States Patent No. 5,454,373 issued October 3, 1995 to Koger et al. describes a rotatable drive shaft of an ultrasound imaging device having a tubular body and a nose member which includes the ultrasound imaging device and connected to the rotatable drive shaft for rotation to obtain different internal views, for example, of a blood vessel. Alternatively, United States Patent No. 5,701,901 issued December 30, 1997 to Lum et al. discusses a pivotable reflector for reflecting an axially-directed ultrasonic beam in a radial direction per Fig. 5.

United States Patent No. 5,997,497 issued December 7, 1999, to Nita et al. discusses use of a foot petal on/off switch for hands-free actuation of a signal generator connected to an ultrasound transducer. United States Patent No. 7,127,401 issued October 24, 2006 to Miller describes remote control of a medical imaging device using speech recognition as well as foot controls. In particular, a surgeon using an ultrasound imaging console may speak a command, for example, "zoom," and a speech recognition processor receives the voiced command for comparison with a look-up table, and, the functionality being available at an associated control console, magnification of a displayed image may be provided to the surgeon. United States Patent Application, US 2002/0065464, published May 30, 2002, describes an imaging device including a wireless mobile unit. Ultrasonic imaging devices and viewing apparatus are large and bulky apparatus. The described imaging device allows an operator to move freely throughout the operating arena, without being tangled within cords and allowing the patient to remain relatively undisturbed while simultaneously allowing the operator full access to the entire patient's body. United States Patent Application, US 2007/0066894, published March 22, 2007, describes a remote wireless control device for an ultrasound machine and method. The remote wireless control device includes a subset of controls present on larger apparatus including a sonogram display. A smaller mobile unit communicates with the larger unit and may be more easily used bed-side than the larger apparatus.

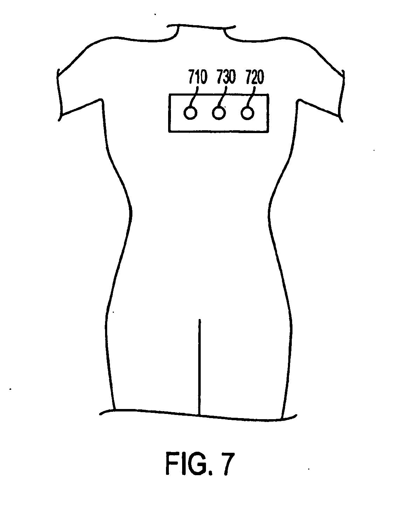

United States Patent No. 6,558,326 issued May 6, 2003 to Pelissier describes a plurality of ultrasound imaging systems connected to a network according to Figure 7 which may be a local area network or a wide area network or combination for communicating with a server and a plurality of clients to provide various services such as report generation, tele-exam, remote control, and patient database administration.

Methods and apparatus for providing three dimensional ultrasonic imaging are known. United States Patent No. 6,572,551 issued June 3, 2003 to Smith et al. describes a three dimensional ultrasound imaging probe configured to be placed inside a body. Published Canadian Patent Application 2 376 103 of Glaser et al. dated January 4, 2002 discusses obtaining three dimensional images by ultrasonic scanning from a minimum of two different positions based on three different send-receive cycles or an arrangement of three acoustic modules and two send-receive cycles, not arranged collinear to each other.

U. S. Patent No. 7,118,531, issued October 10, 2006, to Krill describes an ingestible medical payload carrying capsule with wireless, e.g. ultrasonic, communication to transducers placed on a patient The capsule may deliver medication or contain imaging apparatus such as an optical camera and/or a transducer with a pulse driver for internal acoustic pulse illumination and external high resolution sonogram imaging and detection.

Other dislosures include EP0607490, WO 2001/12068, P1286172, DE3719919,

WO2002/094089, US 6,406,431, US 5,360,005, US 2004/171,935 and US 5,195,519. However, the apparatus and techniques disclosed in these documents all have disadvantages such as high profiles, and difficulty in setting a correct angle.

### SUMMARY OF EMBODIMENTS AND ASPECTS

The invention is defined in the independent claim 1. Preferred embodiments are defined in the dependent claims.

This summary is intended to introduce, in simplified form, a selection of concepts that are further described in the Detailed Description and depicted in the drawings. This summary is not intended to identify key or essential features of the claimed subject matter, nor is it intended to be used as an aid in determining the scope of claimed subject matter. Embodiments and aspects described herein relate generally to embodiments and aspects and methods of use of a wired or wireless transducer element or transducer array that may be remotely controlled to capture multiple image planes at a region of interest of a patient or victim and manipulated or moved to different locations on the skin surface without having to have an operator or large ultrasound system apparatus present at the site of the patient or victim (hereinafter, simply, patient). The patient or victim may be an animal as the embodiments may be used, for, example, in veterinary medicine. Thus, while human patients may be described with respect to various embodiments, the following description and claims are intended to encompass animal species including mammals and, in particular, humans within the term "animal."

In accordance with one embodiment, a transducer array unit or assembly including at least one motor for fixing to a patient's body is wireless and communicates imaging data collected by a typically linear ultrasonic array by wireless means to an external site where collected imaging data is displayed and may be viewed by an operator. One embodiment of a wireless transducer unit is very much like a probe that can be affixed to the body surface and its imaging functions controlled remotely, for example, by wireless radio telecommunication such as WiFi, Wimax, Bluetooth or other radio frequency communication protocol. The wireless communication may also be ultrasonic, infrared or utilize other wireless communication frequencies. Wired communication may be provided via telecommunications including the internet, intranet, twisted pair, optical fiber or coaxial cable or other wired media. For example, pcAnywhere v. 12.1 software, available from Symantec, provides for control of any remote terminal from any other remote terminal and for encrypted two-way communication. The wireless or wired link may be a local or long distance telecommunications link involving satellite transmission. The transducer may be any ultrasound transducer (analog or digital, mechanical, annular, phased array or linear array) and may be single or multi-dimensional. Each transducer array unit may have its own unique identification code which is communicated with each wireless or telecommunications transmission to a host site of ultrasound imaging processing, control and display. The unique code of the remotely manipulatable transducer array unit is used by a host ultrasound imaging remote site to communicate with it. The transducer array unit is, for example, battery powered and self-contained such that it may be worn by a patient with minimal invasion of the patient's privacy. It includes in primary part, ultrasonic transducer circuitry for transmitting and receiving ultrasonic waves, control leads for mode, depth, focus and the like as is known in the art but additionally includes control leads for controlling the movement via one or more motors of the transducer array mounted, for example, on flexible rods of an assembly for movement in two directions, for example, on the skin surface of a patient, transducer rotation and the direction of its transmission (transducer twist). The assembly also may include image data transmission and control data reception circuitry. The transducer assembly thus contains a transducer array that is remotely manipulatable adapted to be fixed to animal (for example, human) skin surface or other location and imaging data collected from the device.

The patient may eat, sleep or, otherwise, function, for example, in or out of their hospital bed while the unit remotely views and transmits imaging data to the remote site. There is no need for a trained person to be at the patient's side. A person untrained in the art of ultrasonic imaging may place the transducer housing(s) including motors and a rod assembly on the body or ask the patient to wear a vest, girdle, hat or other article of clothing containing the transducer assemblies. On the other hand, a trained observer may communicate with the assembly from a distance of a few feet to thousands of miles by an appropriate communications channel to remotely control the imaging unit and obtain images therefrom. The image data may be displayed at the remote site as a still image or a moving image (4D) of a three dimensional space which may be stereoscopic. The assembly of this embodiment fixed to the patient's body may comprise an array of transducers that may be remotely controlled to rotate from one position to another, either clock-wise or counter-clock-wise, to obtain a different planar view of the body part under analysis. The shape of the ultrasound transducer assembly may be round or square or rectangular for providing multi-planar images. The wireless transducer unit may function with a three dimensional imaging system allowing stereotactic observation and remote/robotic operation of devices delivered through or in conjunction with the unit as will be further described below. The transducer array may be fixed to a rotor or rotors of one or more motors and the rotor assembly and transceiver circuitry housed within a housing having for example a cylindrical shape with one side intended to be facing the patient's body. The flat side facing the body may have a layer of body impedance matching material complimentary to any gel application. A micromotor and optional associated gear assembly may incrementally rotate the transducer array or element, for example, via the associated rotor and optional gear assembly through a range of π rad (180 degrees) or linearly move the transducer in x or y or both directions. Transducer position may be remotely determined and stored in local or remote memory and/or displayed at the external remote control site. The size of the footprint of a housing for a remotely manipulatable transducer assembly on a patient's body surface may be as small as 1 cm or as large as several centimeters in diameter (or length/width). A typical operating frequency of the ultrasonic transducer array or element may be between 20 kHz and 400 MHz depending on the clinical application. Different frequency ranges of one ore more ultrasound transducers can be used for different purposes and provide different beneficial results. Frequencies in the lower range, for example, below 1 MHz, and particularly in the 100-200 KHz range, can be used, for example, to provide heat therapy or to treat conditions such as blood clots. Frequencies above 1 MHz can be used to provide imaging where the higher the frequency, the greater the resolution possible but the lesser the depth reachable in the human body below the transducer. As indicated above, reception at harmonics of the transmitted ultrasound frequency may improve imaging at depths, for example, in imaging obese animals. Also, smaller structures may be imaged in a range of frequencies up to 200 or 400 MHz. In addition to rotation, a linear transducer array may be adaptably mounted to a rotor shaft so that it may also redirect output sound waves within a range of π rad (180 degrees) of twist within the patient's body at the given angle of rotation. A first transducer array may cooperate with a second or plurality of similar transducer array units situated inches away from the first array as a transmitter while the second device operates as a receiver and vice versa and for three dimensional image, still and motion, capture. The first and second transducer array units may separately provide image data of the same region of interest to a remote workstation for three dimensional image capture. The rotatable transducer embodiment may, for example, be of circular, convex, curvilinear, skin surface conforming or cylindrical shape and may be affixed to the body by a broad securing material that may be elastic, adhesive or non-adhesive, such as a band or bandage of cloth, elastic or other fiber. No operator need be attendant at the patient site. Imaging data may be converted from analog to digital format, if necessary, and compressed before it is transmitted in accordance with well known standards to conserve transmission bandwidth. Typical ultrasonic imaging bandwidth requirements should be on the order of 1 MHz especially if known image compression algorithms are utilized at the remotely controlled unit prior to imaging data transmission. If high levels of resolution are required, the data transmission bandwidth may exceed 5 MHz or, if low resolution is permitted, a 100kHz bandwidth may suffice.

In yet another embodiment, the patient may be an out-patient and wear a battery-powered transducer array assembly apparatus including at least one motor that may be remotely monitored and manipulated via a wired or wireless telecommunications channel or by the out-patient via a cord to a remote control. Image data may be collected and stored locally in removable or accessible memory over time. The out-patient may visit their doctor and the memory contents unloaded rather than be remotely transmitted. On the other hand, the memory, when it reaches a predetermined fill capacity may transmit its contents via the transceiver to a remote workstation for analysis or signal the wearer to do so. If a remote workstation operator sees an extraordinary condition in ultrasound imagery of an out-patient, the out-patient may be warned by the operator's triggering a vibration or other alarm of such condition via a telecommunications or wireless link, for example, to indicate that the out-patient must see their doctor immediately.

The operator may continuously monitor, for example, from the remote site for signs of patient difficulty. For example, the development of a blood clot or other serious condition may be viewed remotely if the device is used in conjunction with, for example, a knee replacement operation. This same operator may be connected to a plurality of patients via a server and work from a client workstation of the server.

The out-patient may control the transducer array or element themselves, for example, to deliver therapeutic ultrasound waves to a region of interest to them and so manipulate the transducer array under rotation or twist motor control to change a direction of propagation of sound waves different from the same or a different transducer array used for imaging by a remote operator. There thus may be provided two different transducer arrays (therapeutic and imaging) operable at different frequency ranges and different remotely manipulatable motor control for the two arrays (imaging controlled via a remote workstation and therapeutic operation controlled locally by the patient) or a common motor control of both arrays in an alternative embodiment with shared control and priority of control given to the remote workstation.

In another alternative embodiment, the transducer array unit may be formed as a square or a rectangle and the linear transducer array in addition to rotation, twist or direction of sound wave transmission may move under remote motor control in a lengthwise or widthwise direction from one end of a square, cylindrical or rectangular shaped housing or one conforming to the shape of a body cavity to the other end. In another embodiment as described above, the array may move in two perpendicular directions, for example, in an x or y axis direction on the body surface and not be permitted to rotate or twist. Such a device may comprise a single transducer element or a transducer array such as a linear array. On the other hand, a transducer array, for example, contained in a square or rectangular housing may also be rotatable to a predetermined angle of rotation by remote control at each incremental lengthwise or widthwise position and/or twisted. For example, such a transducer array unit may be used to monitor a fetus within a patient as it moves within the abdominal cavity. One unit may be fixed to a female patient's body and be manipulated alone or in conjunction with another or plural remotely manipulatable transducer units affixed to the female patient's body.

In an alternative embodiment and in conjunction with an imaging catheter as described in my co-pending United States patent application Serial No. 11/782,991, filed July 25, 2007, one or more remotely manipulatable transducer unit assemblies may be used together with the imaging catheter to provide additional imaging of a minimally invasive heart operation or other procedure being performed on a patient in an operating arena. In deed, visualization of any body part is possible including the heart, liver, kidney, brain, prostrate, vagina, uterus, breast, any vascular structure, gland (such as the thyroid), extremity (knee replacement) or other body part to be monitored. For example, the remotely manipulatable wireless transducer unit may facilitate any intervention requiring ultrasound guidance including but not limited to entry into various body spaces such as a pleural, vaginal, skull, peritoneal and pericardial space thus allowing therapy delivery, intervention, placement of devices such as pacemakers, IUD's or medicine pumps and diagnostics. One skin surface mounted transducer may cooperate with a second body cavity transducer having a linear motor for lengthwise movement along its linear axis, and other motors for rotational movement and twist movement of the transducer. For example, such a transducer may have a cylindrical or other shape conforming to the given cavity and be introduced into the rectum, vagina, nasal cavity, ear cavity, oral cavity or other body cavity or be similar to the known Krill payload that a patient has ingested to obtain further imaging of a region of interest and/or three dimensional imaging. In addition to an imaging catheter, one or more remotely manipulatable wireless transducer array units may be used with another interventional, therapeutic or diagnostic system such as a biopsy forceps, a drainage catheter, a pressure monitoring system, a suture application system, a therapy delivery system or other interventional, therapeutic or diagnostic system known in the art.

In yet another embodiment, the remotely manipulatable transducer array unit may deliver ultrasound energy for therapeutic rather than imaging purposes, for example, to specific locations on or under the skin surface or within the body. During an interventional or a therapeutic procedure, the interventional procedure requiring intermittent ultrasound monitoring such as surgery or cardiac catheterization, the transducer element or transducer array assembly can remain on the body during the entire procedure and the imaging and/or therapeutic treatment performed as and when needed by the remote operator. The remote operator may communicate with a surgeon or other operating room personnel by telecommunications to, for example, report that a medicine pump has been properly placed and is operating, for example, via a headset worn by the surgeon or other operating room personnel within an operating room.

The remotely manipulatable wireless transducer array unit communicates with a remote workstation, for example, via a central server serving a plurality of client workstations. Each remote workstation may include at least one display and a user interface screen for not only viewing an imaged area but, for example, a compartmentalized image area of a plurality of image displays of a region of interest and additionally present a user interface providing, for example, time of day, date, rotational degree and other control feedback in conjunction with usage of a control device such as a trackball or mouse. Other known controls such as on/off, frequency range, depth of field, focal length, sweep speed, magnification, gain, focus, contrast, brightness, selection of color Doppler imaging versus conventional gray scale, measurement of Doppler velocity and the like make be provided via conventional buttons, knobs or monitor screen controls. The workstation may comprise one or a plurality of displays of the transmitted image of a region of interest including a moving image three dimensional (4D) display or plural displays of multiple planes or a display showing manipulation of the ultrasound transducer element within the boundaries of a housing as placed on a patient or in a patient cavity and/or a display of operating parameters such as the coordinates of location of the transducer, its angle of rotation and its twist or angle of sound transmission. If plural transducer units are utilized, the single workstation may provide additional displays for each remotely controlled and manipulatable transducer unit.

A first remote workstation may communicate with a second remote workstation by wired or wireless means, for example, via a central server, and the second remote workstation may serve as a back-up to the first remote workstation in another embodiment. An operating room can only efficiently contain so many people assisting a surgeon and so much equipment. For example, the primary area of use of a wired or wireless remote transducer unit may be within a fluoroscopy suite or an operating room such that one or more remotely manipulatable wired or wireless transducer assemblies communicates with a primary workstation and/or a secondary remote workstation outside the suite or room. In such a situation, each of the primary and secondary workstations are uniquely identified and a central server serving both workstations may be uniquely identified as are the wireless transducer units uniquely identified and only one workstation is able to remotely control one wireless remote transducer unit at a time while the other workstation may be afforded monitoring privileges. As suggested above, a control operator of a workstation may communicate with the surgeon by means of a headset to answer questions a surgeon or other operating room personnel may have as a yes or no or advise of a successful procedure.

These and other embodiments and aspects will now be described with reference to the drawings and the detailed description to follow.

### BRIEF DESCRIPTION OF THE DRAWINGS

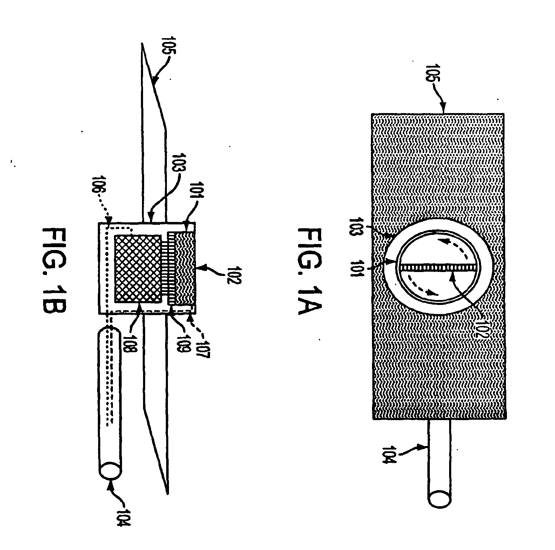

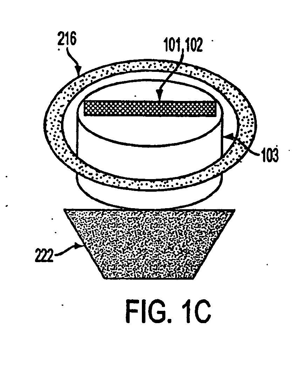

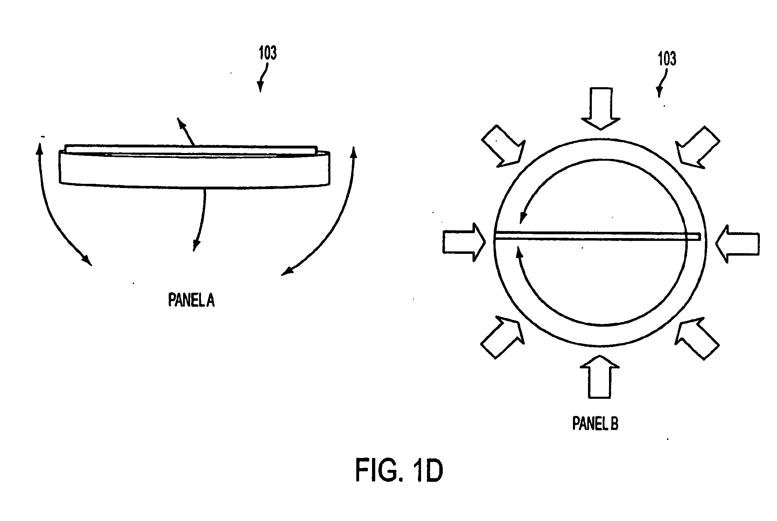

Figure 1 comprises a top view (FIG. 1A) and side view (FIG. 1B) of a first plurality of embodiments and aspects of a multi-plane transducer assembly unit comprising a rotatable linear array of transducer elements including a housing for mounting by securing material to a body of, for example, a patient or victim, which may be controllably rotated and otherwise controlled by wired or wireless signals remotely from the patient without an operator needing to be proximate to the body to manipulate or control the transducer elements or the housing; Figure 1C provides details of rotational and tilt or twist motor movement of a transducer element or array; Figure 1D provides further details of rotation and seesaw or tilt/twist movement to capture multiple image planes per panel A (side profile) and B (en face view)

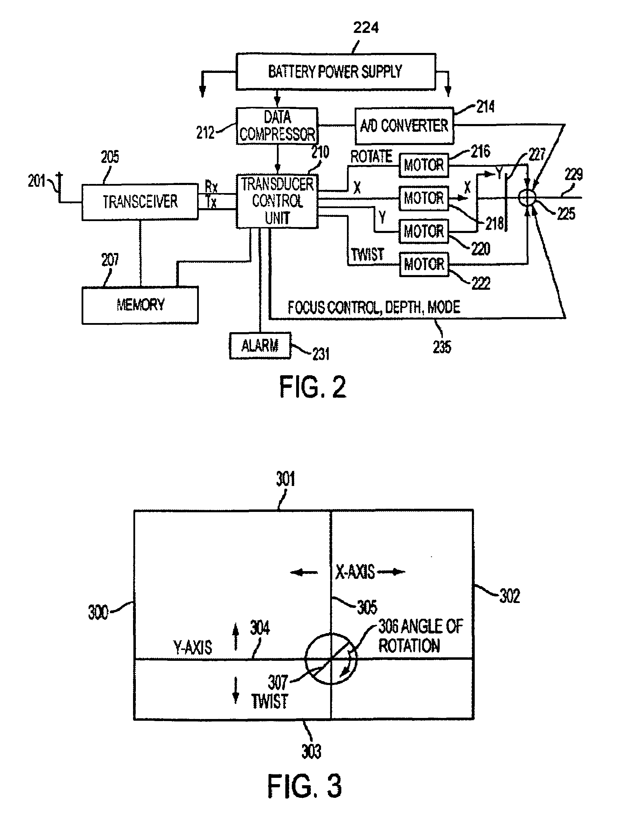

Figure 2 provides a schematic block diagram for embodiments and aspects of a device as shown in Figure 1 which may be wireless (or wired) and further including a wireless transceiver in addition to a transducer control unit Also shown are a battery power supply, one motor for rotating a linear transducer element array, a motor for moving the array in each of perpendicular X and Y directions and a motor for twisting the array, the linear transducer element array and analog to digital circuitry (if necessary) for converting collected image data to digital form and a data compressor for compressing data for digital transmission via the transceiver.
Figure 3 provides an overview of a mechanical arrangement of, for example, flexible or body shape conforming rods to be contained within a housing of embodiments of a transducer assembly unit for manipulating a transducer or linear transducer array in two perpendicular directions, for example, along an x axis and a y axis, to provide an angle of rotation to permit multiple image planes and a twist angle to redirect a sound wave emitted by a transducer or linear array of transducer elements whereby it is envisioned that a footprint on a patient body surface is rectangular or square and relates to the embodiments and circuits of Figures 1 and 2.

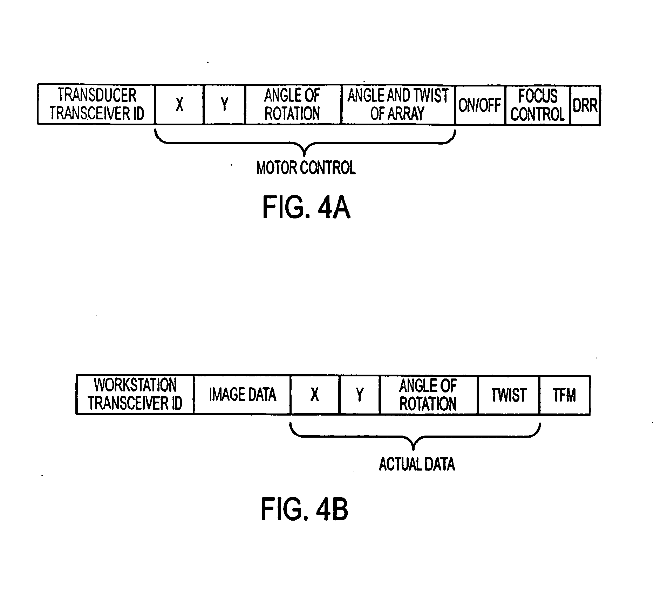

Figure 4A provides an exemplary signal content format for providing motor control of a remotely manipulatable transducer or transducer array of Figure 1, 2 or 3 in a direction from a workstation to a remote wired or wireless transducer including a unique transducer transceiver identifier if wireless or telecommunications transmission is utilized. The format also provides for known and herein introduced control such as on/off, focus, focal length, frequency of operation, brightness, contrast, depth of field (related to frequency), sweep speed, contrast, magnification, mode such as selection of color Doppler imaging versus conventional gray scale, measurement of Doppler velocity, time and date and the like. Moreover, image data, for example, recorded in known JPEG or AVI format may be transmitted as will be explained with reference to FIG. 4B and subsequently received at the transceiver for verification with transmitted data stored in memory 207.
Figure 4B provides an exemplary signal content format for providing a reply signal from a transducer or transducer array of Figure 1, 2 or 3 in a direction from a remote wired or wireless transducer including a unique workstation identifier if wireless or telecommunications transmission is utilized toward the workstation or an associated server. The depicted format provides for feedback of actual location data of the position of the transducer or transducer array, time and date of image data (not shown), as well as image data collected for that location and time and date.
Figure 5A provides a plurality of suggested locations on a human body for locating a remotely manipulatable transducer or transducer array assembly whereby the assemblies may comprise devices and elastic or adhesive material similar materials used for fixing electrodes used in electrocardiography, banded adhesive attachment or bandage or banding of the manipulatable transducer or transducer array housing to a human patient's body. FIG. 5A does not show suitable body cavities such as nasal, oral, rectal, vaginal, ear or other cavities in a human body in which a remotely controlled wired or wireless transducer or transducer array assembly may be inserted and where there may be no need for any securing material. FIG. 5B shows a single patch comprising transducers for heart, spleen, kidney and liver monitoring. Also, while FIG. 5B could be worn as an article of clothing, FIG. 5B does not show clothing such as a vest, girdle or hat constructed of suitable material which may provide one or a plurality of transducer assemblies constructed into the clothing as per FIG.'s 1-5B which may all transmit and receive a signal from a shared antenna 201 built into the article of clothing. The wearer may prepare an imaging site by applying suitable ultrasound gel to the areas on the clothing containing the assemblies or, alternatively, apply the gel to the body before putting on the clothing.

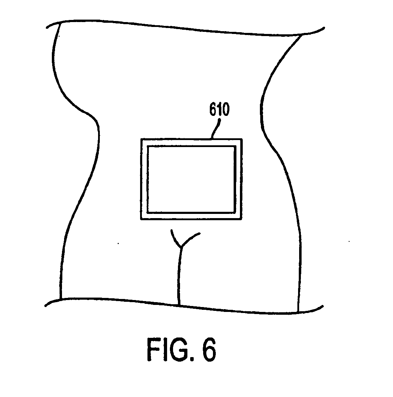

Figure 6 provides an exemplary application of a manipulatable transducer array that may be worn by a pregnant female in the abdominal area for remotely monitoring a fetus for, for example, extraordinary conditions. Additional transducers may be mounted at other external or within internal body locations for providing three dimensional and alternative views of the fetus.
Figure 7 shows an arrangement for pericardial activity and possibly surgery including first and second manipulatable transducer units 710, 720 mounted to either side of, for example, an image guided catheter 730 surgical location as one example of an application for minimally invasive heart surgery. Imaging transducer 710 may cooperate with imaging transducer 720 or image guided catheter 730 to provide a three dimensional image of the heart and pericardial cavity and monitor the movement of image guided catheter toward an operating area of the heart or tools for delivery via the image guided catheter 730.

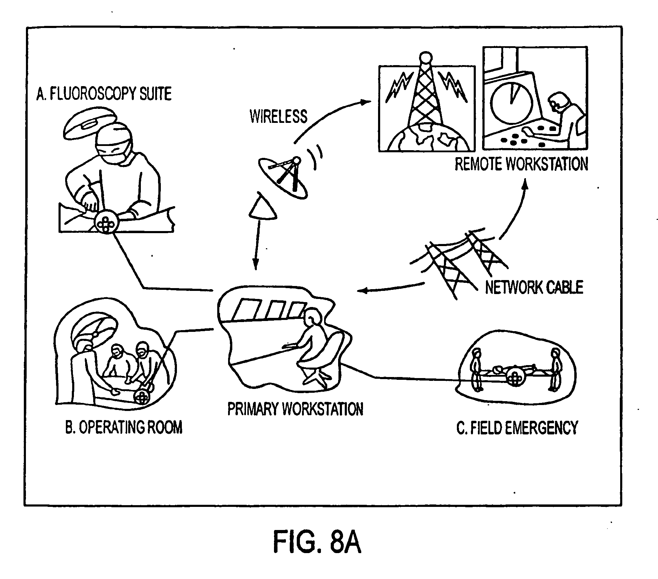

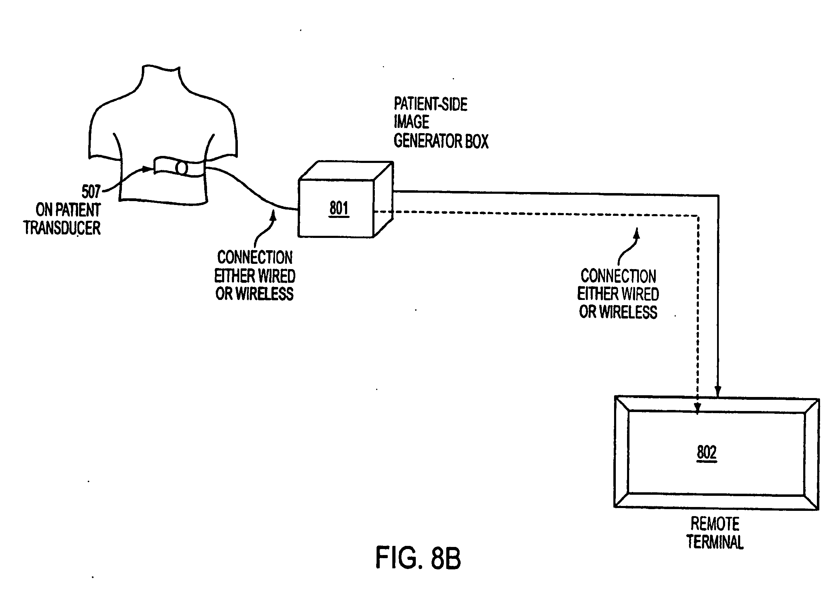

Figure 8A shows a workflow scenario whereby the primary workstation or server and plurality of workstations is remote from a fluoroscopy suite, operating room and the like and, if necessary, shielded from adverse impacts of radiation such as magnetic resonance. FIG. 8B provides details of wired/wireless connections between a patient, an image generator and a remote terminal.

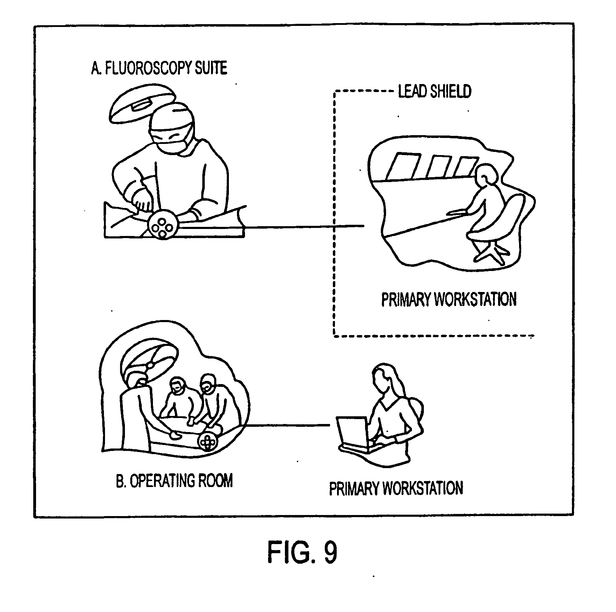

Figure 9 shows another workflow scenario for describing, for example, remote field emergency use, operating room, fluoroscopy suite use and primary and alternative workstations connected by a wireless or wired telecommunications network where a primary workstation having a plurality of displays is shielded from adverse impacts of radiation.

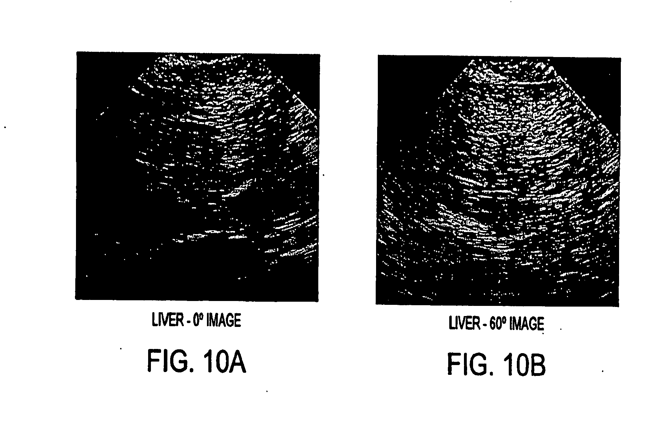

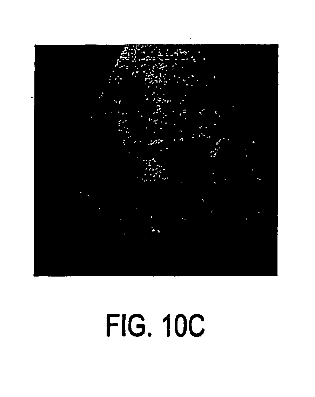

Figure 10 provides views of a liver obtained via an assembly embodiment constructed similarly to that depicted in Figure 1 and affixed to a skin surface by stretchable adhesive tape wherein Figure 10(a) represents an image at 0 rad rotation, Figure 10(b) represents π/3 rad (60°) rotation as examples of image planes; Figure 10(c) represents a three dimensional color Doppler image shown in grey scale.

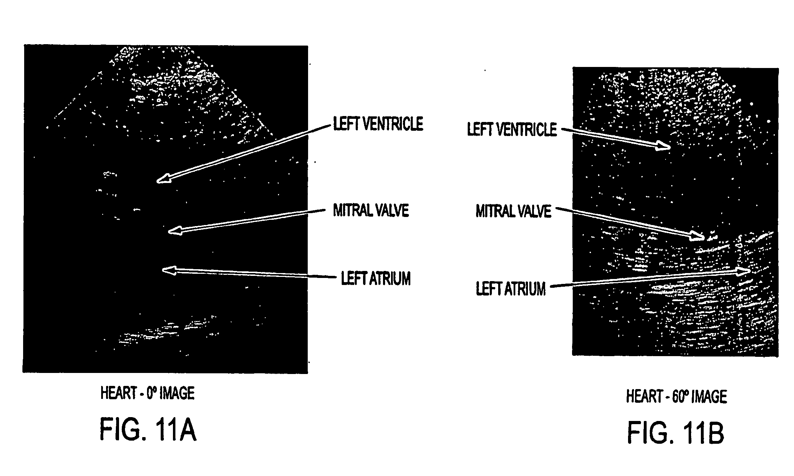

Figure 11 provides views of a heart obtained via an assembly embodiment constructed similarly to that depicted in Figure 1 wherein Figure 11 (a) represents an image taken at 0 rad (0°) rotation and Figure 11(b) represents an image taken at π/3 rad (60°) rotation.

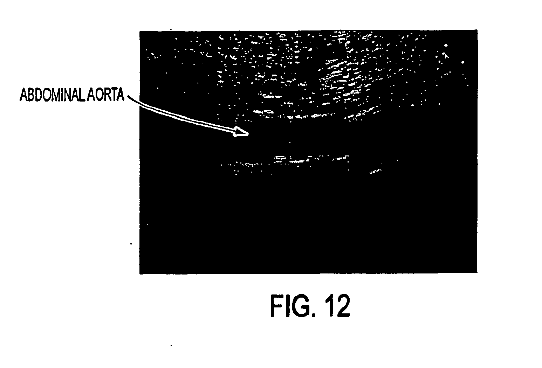

Figure 12 provides a view of the abdominal aorta obtained via an assembly embodiment constructed similarly to that depicted in Figure 1.

### DETAILED DESCRIPTION OF THE EMBODIMENTS

Figure 1 comprises a top view (FIG. IA) and side view (FIG. IB) of a first plurality of embodiments and aspects of a multi-plane transducer assembly unit comprising a rotatable linear array 102 of transducer elements including a housing adapted for forming a beam of ultrasonic energy and receiving a reflected beam at primary and harmonics of the transmitted frequency. Figure 1C provides details of rotational and tilt or twist motor movement of a transducer element or array 101, 102 of, for example, piezocomposite or crystal material of housing 103 via motors 216, 222. Vermon of France's US subsidiary, Vermon, USA of Lewistown, PA, provides 1-3 piezoelectric composite transducers that may be customized for a particular purpose. Figure ID provides further details of rotation and seesaw or tilt/twist movement to capture multiple image planes per panel A (side profile) and B (en face view) of housing 103. The housing 103 comprising transducer elements 101 and/or 102 may be mounted by securing material 105 to an animal (human) body of, for example, a patient or victim (not shown). The transducer array or element 101, 102 may be remotely controllably rotated and otherwise remotely controlled by wired (cable 104) or wireless signals transmitted toward the transducer 101 or array 102 from a remote workstation (not shown). An operator need not be proximate the patient's body to manipulate or control the transducer elements 101, 103 or housing 103. Similar reference numbers will be used throughout the detailed description to refer to similar elements wherein the first number of the reference number denotes the figure in which an element first appears. Transducer 101 may comprise a single transducer element for ultrasonic transmission and reception of reflected sound waves or, for example, a linear or other shaped array 102 of transducer elements mounted, for example, in a circular manner from a top perspective as a diameter of the circle or at the center of the circle comprising housing 103. An arrow indicates an angle of rotation in a clockwise or counter-clockwise direction of the transducer element 101 or a transducer array 102 within housing 103. Typically, an angle of rotation of π rad (180 degrees) when used with a transducer array 102 will permit the collection of a plurality of image planes, for example, of the heart over which the transducer array 102 within housing 103 may be located and fixed to the body surface by a fixing adhesive band or non-adhesive wrap 105, in this case, a cylindrical housing 103 as seen from top and side views forming a circular footprint on the body surface. The housing 103 is fixed to the surface, for example, of a human body, for example, in a position at the center of the chest to monitor the heart immediately below. The top surface of the side view shows transducer 101 or transducer array 102 which may rotate within the housing 103. The top surface of housing 103 intended to be fixed to a patient contains an impedance matching substance which may be complimentary to the application of a suitable impedance matching gel. Fastening or securing material 105 is shown in top and side views for fixing the housing 103, for example, to a human body skin surface with the transducer/impedance matching surface facing the human body surface. The securing material 105 may be a band or elastic band that wraps around the body or, in an alternative embodiment, an article of clothing. Within the housing 103 is also contained at least one motor, in the embodiment of Figure IB, for example, a motor 108 for rotating a transducer element or linear array 102. Also located within the housing 103, for example, in the vicinity of the motor may be a wireless transceiver and antenna (not shown; see, for example, Figure 2), a transducer control unit 210, and other circuitry as necessary for receiving and processing motor control signals and other known control signals such as on/off, mode of operation (such as color Doppler versus conventional gray scale), depth of field, brightness, contrast, focal length, sweep speed, magnification, frequency of operation, focus, focal length, Doppler velocity measurement and the like. Also, not shown is a battery or power system for powering the motors and circuits requiring power. Alternatively to a wireless device, housing 103 may have a control cable or wire 104 for transducer output control, providing power, motor control and the like.

Cable 104 may lead to a workstation console, preferably remote from a patient bedside or to a server communicating with a plurality of workstations and operate in a similar manner to known cables used with devices such as a Toshiba PowerVision ™ ultrasound machine, the difference being that the depicted cable 104 further includes a rotation, twist, linear direction or other motor control lead or leads or a data line of such cable further incorporates rotation, direction and twist motor control data in a serial data stream on a single data lead. Alternatively, another manufacturer of ultrasound workstations is Analogic Corporation and, in particular, B-K Medical A/S of Herlev, Denmark. Per FIG. 1B, cable 104 may include at least motor control wiring 106 connected to rotation motor 108 for control and power purposes. Cable 104 may further comprise transducer wiring 107 for power, transducer control and image collection purposes.

As will be described herein and as illustrated by FIG. 1C and FIG. 2, further motors 108 such as motor 222 may be provided for twisting linear array 102 to permit a different direction of sound wave emission and/or reception of reflected sound waves. Still further motors 218, 220 provide two directions, for example, lengthwise and widthwise (x and y) axis movement in the plane of the human body surface and according to how a rectangularly shaped housing 103 constructed of flexible or body surface conforming rods per FIG. 3 is placed on the body, i.e. an x and y axis are considered in relation to the housing 103. The housing 103 may be mounted at an angle (see, for example, manner of fixation 507 or 502 of Figure 5A to the human body) or other manner most comfortable for the patient, yet effective. Motor 108 may comprise an optional gear assembly 109 (FIG. 1B) for more accurate, for example, incremental movement of transducer array 102. Motor 108 is preferably a micro or miniature linear motor known in the art for turning a rotor for twisting an array 102 and associated optional gear assembly 109 for rotating the coupled transducer element 101 or linear array 102 at incremental steps such as one degree steps from a vertical or horizontal orientation (vertical shown) through a 180 degree range - clockwise or counterclockwise. In this manner, a linear array 102 may capture up to 180 different planes of view of, for example, a heart under observation, and a moving three dimensional (4D) view may be constructed using known software data analysis processes. Of course, the three dimensional analysis is improved and made stereoscopic if pairs (or more than two arrays) of devices at different observation locations according to Figure 1 are used as will be described in conjunction with a discussion of Figure 7. A transceiver 205 (FIG. 2) or a cable 104 may report the actual rotation position of the transducer array 102 to a remote workstation (not shown) as a value, for example, between 0 and π rad (0-180 degrees). The physical location of the transducer 225 at a given time may be calculated by detecting a known body part, such as a heart, and from the x, y, depth, rotation, twist, frequency of operation and other control data received at the workstation from the transceiver 205 calculate the physical position of a given housing 103 on the skin surface. In an alternative embodiment, the electronic circuit of FIG. 2 may comprise an input keyboard or selector for selecting one of a plurality of locations on the body, such as chest or abdomen, and/or a target organ, such as one of two lungs or the heart.

Typical sizes for a cylindrical transducer housing 103 as shown in Figure 1 may be from 1 cm in diameter to 3 cm in diameter. The height of the cylindrical housing may be similar or less than 1.5 cm. The size of a housing 103 is directly related to the components it contains. In embodiments intended to be placed in body cavities, the housing 103 may be sized to conform to the cavity. Certainly, the trend in the electronic arts including mechanical motor arts is toward further miniaturization and integration. Consequently, notwithstanding the given dimensions, it is not inconceivable that a housing 103, for example, may be reduced in height or thickness to the range of millimeters or even micrometers within the next twenty years. The housing may support the movement of and OCT, infrared or other medical imaging device known in the art. The size of the footprint on the human body or whether the device is used internally in a body cavity may relate to the particular application and not necessarily to the sizes suggested above for housing 103.

If a housing 103 has a rectangular shape, for example, for observation of a fetus (see Figure 6), its shape may be on the order of size of 10 cm by 12 cm, in which case, linear motors are provided for two directions, for example, x and y axis manipulation of the transducer array in these larger dimensions in addition to rotation and twist. Referring briefly to Figure 3, the surface proximate to the body of a housing 103 may comprise a rectangular shape and linear motors may move small rods carrying, for example, a transducer array to a particular x, y coordinate ranging from 0 to 10 cm in one direction to 0 to 12 cm in the other direction within its footprint on the body surface in incremental steps, for example of 5 mm. In a further embodiment as described above, a motor 222, 307 may be provided and mounted to twist a linear array as well as provide an incremental angle of rotation, again, within a range of 0 to π rad (0-180 degrees) with a default position at π/2 rad (90 degrees), or directly pointing sound waves into the human body (see FIG. 1C). In an alternative application, for example, to image a human heart, the x and y motors 218 and 220 may be actuated to improve imaging by moving a location of transducer or transducer array 225 to another location removed from a partially obstructed ultrasound path or one including undesired reflections, for example, caused by an implanted device or an invasive device used in the vicinity of the heart region of interest.

Figure 2 provides a schematic block diagram for embodiments and aspects of a wireless device as shown in Figure 1 including a transceiver 205 (which may be a wireless telecommunications transceiver), a transducer control unit 210 which may comprise a microcomputer, a battery 224, at least one motor for rotating a transducer element array, for example, a linear array, the transducer element array 225 and analog to digital circuitry 214 (if necessary) for converting collected image data to digital form. There is also provided a data compressor 212 for compressing data for transmission via the transceiver 205 and antenna 201 or cable 104. In one embodiment, the analog to digital converter may be eliminated as data may be collected in digital form from transducer array 225. In Figure 2, a wireless embodiment of a remotely manipulatable ultrasound transducer is assumed. Battery supply unit 224 is preferably a rechargeable lithium battery known in the art that powers all circuits requiring power within a housing 103 (not shown in FIG. 2). Controller 210 has associated program and data storage memory 207 and a clock (not shown) for synchronizing pulsing transducers, image data collection and data transmission to a workstation, for example.

Transceiver 205 is an alternative data transceiver to a control and data cable 104 for transmitting and receiving information and may receive and transmit a digital data signal generally in keeping with Figures 4A and 4B via antenna 201. While these figures depict what may be construed as a serial data stream, the depicted data may be sent in parallel or serial format and in any order including the order shown. Moreover, the depicted data transmitted in each direction may be supplemented by other known control or imaging data and other unit identification data such as server address data, the server for serving a plurality of workstations. Known telecommunications protocols may be utilized if the transceiver 205 or cable 104 transmits and receives by radio frequency signal such as WiFi, Bluetooth, Wimax and the like for a wireless local area network or optical signal via a dual mode optical fiber 104. Telecommunications cable 104 may be twisted pair, coaxial cable as well as optical fiber and may support POTS, intranet and internet telecommunications as well as other known forms of telecommunications or cable television channel. As is known in the art, infrared and ultrasound may be used as well as other light frequencies than infrared as may transmission in the microwave band. On the other hand, light waves are typically incapable of penetrating through walls and require a line of site transmission path. Yet, by way of example, light wave transmission is feasible; for example, a light wave transceiver connected to a workstation may be mounted, for example, in the ceiling of an operating arena and a unit mounted to a patient facing upward may communicate with the ceiling mounted unit in a line of sight. As described above, since a cable 104 may provide a direct link to a remote workstation or to a server communicating with a remote workstation, cable 104 need not necessarily transmit data uniquely indicative of a given transmitter, transducer, workstation or server because the cable 104 may comprise a direct, switched or multiplexed link or digital channel between known devices. Alternatively, the data of FIG. 4A and 4B may additionally comprise a unique server address if a server is host to a plurality of client workstations or secondary back-up workstations. Moreover, the workstation ID of FIG. 4B may be a primary workstation, and the data signal further comprise an address for one or more secondary workstations. If any other device is connected to cable 104, then addressing using a unique address (or telephone number) or other identifier should be used for a connected device. Transceiver 205 may receive a data signal from a workstation, demodulate the signal and output a demodulated baseband data signal including data per Figure 4a to transducer controller 210 which may be a microprocessor, application specific integrated circuit or other control circuit which may be designed and fabricated in a manner well known in the art. Transducer controller 210 may run a real time clock and date program synchronized periodically with a real time clock at an associated workstation. In the other direction of transmission, the transceiver 205 may receive image data for one or more planes or sequential images and other signal including actual position data (for example, x and y coordinates, magnification, depth, rotation angle, twist angle, real time and date and the like) per Figure 4B from transducer controller 210 for temporary storage in memory 207 or for wired or wireless transmission to a uniquely identified/addressed remote workstation.

Following the path of a received signal at antenna 205, the received signal may be received at radio frequency at transceiver 205 (or via cable 104 at radio frequency, optical frequency or baseband), demodulated, if necessary, and a Rx data output signal passed to controller 210 for processing. Controller 210 authenticates the signal as directed to it by means of the transmitted unique transducer identification code of Figure 4a. In addition, the signal may require processing in accordance with well known protocols for decompression, decryption, parity and other data error detection and correction algorithms and the like (not shown). In one embodiment, for example, for multi-planar imaging purposes, the transducer array 225 is linear and may be rotated. A rotate signal which may indicate an angle between 0 and π rad (0-180 degrees) in incremental steps of, for example, 0.02 to 0.09 rad (1 to 5 degrees) can indicate rotation in a clockwise or counterclockwise direction or indicate an angle to which the transducer array or element is to be rotated (for example, from π/2 rad (90 degrees), actual present position, to π/3 rad (120 degrees), desired position) is received and passed to linear motor 216 having a rotor for rotation using, possibly, an optional gear assembly 109 for turning the linear array 102 to a desired angle of rotation. A current position of the transducer 225 may be stored in memory 207 as actual motor control data per FIG. 4B for association with image and TFM data as will be described further herein.

In an alternative embodiment, for example, for therapeutic purposes, a direction of sound wave propagation, frequency of transducer operation (if variable), depth (dependent on frequency) and the like signal are received and reported to actuate twist motor 222 to a desired angle of twist in addition to a desired angle of rotation via motor 216 to, for example, deliver a therapeutic sound wave to a given body organ or sub-tissue layer at a given transmitted depth, for example, represented by a sound wave power level, within the patient's body from the transducer 102, 225 and temporarily stored in memory 207. In an embodiment paired with another unit, the angle of twist and rotation may be synchronized so that one transducer array 102 may cooperate with another transducer array as sound wave transmitter and sound wave receiver for together providing image data either individually or together. Also, a therapeutic transducer (operating at a lower ultrasound frequency range) and an imaging transducer (operating within a higher ultrasound frequency range) may be mounted to the same movement system 300 per FIG 3 within the same or different housings 103.

In a further alternative embodiment, the transducer array 102 or transducer element may be manipulated in two directions, perpendicular to one another, along the patient's body surface, denoted an x direction and a perpendicular y direction or axis as shown in Figure 3. If used inside a body cavity such as a nasal, oral, ear or other body cavity, a cylindrically shaped housing may contain a transducer which may rotate 2π rad (360°), twist π rad (180°) and be moved by separate motors along the length of the cylindrical housing 103 shaped for the cavity to different lengthwise, axial positions to capture any number of side views in practically any direction of ultrasound propagation. Beamforming may be additionally used to form ultrasonic waves for a particular purpose. For example, a transducer positioned in the ear may obtain imaging of the brain, inner ear, nasal passages and other portions of the skull depending on frequency range and transducer pulsing delay as known in the art or to apply therapeutic treatment. In the oral cavity, for example, one may ask the patient to attach or hold a flexible patch with their tongue a transducer assembly with x, y, twist and rotation made of flexible rods 300-305 per FIG. 2 conforming to the roof of the mouth. The transceiver 205 outputs such transducer control data to controller 210 which then actuates motors 218 for x axis movement and 220 for y axis movement, 216 for rotation and 222 for twist of transducer element or transducer array 102, 225 as shown in Figure 3. Also shown in Figure 2 are x, y axis 227, 229 which are controlled by motors 218, 220 corresponding to x and y axis 305, 304 of FIG. 3. When arriving at the x, y position of interest, the transducer 102, 225 may be rotated or twisted or rotation and/or twisting/rotation may occur en route to the x, y position of interest. Feedback to the remote workstation may be provided via actual data stored in memory 207 indicating all parameter values of interest, on/off, focus level, depth, magnification, ultrasound frequency, x axis, y axis, angle of rotation and angle of twist (most of which are shown in Figures 4A and 4B) as well as image data and workstation address or identification.

Also, controller 210 may be in receipt of motor control, off/on, focus control, mode, magnification, frequency of operation, depth and other control data which is passed to transducer 102, 225 for proper operation, for example, to regulate the amount of power delivered to transducers for sound wave emission or for focusing the array. This control lead or collection of leads is shown as data line 235. If more than one transducer is provided for, for example, simultaneous imaging and therapeutic purposes, then, a selection bit for selecting one or the other transducer or array 225 may be included in the data of FIG. 4A.

The output of transducer array 102, 225 may be raw image (reflected sound wave) data similar to that obtained by a hand-held transducer array known in the art. It may be in analog form and provided to an A/D converter 214 (optional) for sampling at an appropriate sampling level, for example, depending on desired image resolution or in digital form. The data signal output of A/D converter 214 (optional) may be further compressed at data compressor 212 prior to formatting at controller 210 for transmission at transceiver 205 and/or storage at memory 207, for example, according to FIG. 4B. These circuits 214 and 212 are shown as separate circuits but may, together with controller 210 be in the form of a single application specific integrated circuit (ASIC) or provided as separate circuits. Memory 207 may be on board a microprocessor chip or provided separately. In one embodiment, memory 207 may comprise a removable memory for uploading, for example, imaging, actual position and TFM data collected over time to a device for telecommunications transmission. The image and other data prior to transmission or for long term storage may be temporarily or more permanently stored in memory 207. Similarly, memory 207 may be utilized for temporarily storing control data as received from transceiver 205 and prior to being operated on by controller 210. In one embodiment, there may be no data transmission via cable or wireless means; data may be transferred, for example, via a removable memory 207.

Image and associated position data and the like for a given image along with time of day and date may be stored in a fanny pack or personal remote control device worn or otherwise carried by the patient. This assumes a time of day and date clock associated with controller 210 or the time and day may be periodically updated via a transmission to the unit of Figure 2. In, for example, a therapeutic embodiment of a remotely manipulatable transducer array, the patient wearing or carrying one or more assemblies may control delivery of therapeutic sound waves via a transducer array 102 and control the direction and depth of transmission. For example, ultrasound has been found to assist in relieving arthritis and other pain, for example, in a hip, shoulder, knee or other joint.

In one embodiment where the circuitry and motors are contained in a housing and in accordance with Figures 1, 2, 3 and 4, the housing is worn by a person, the person may be remotely observed as they go about their daily routine. For example, a remotely manipulatable ultrasound transducer array 102, 225 located so as to monitor a major organ may detect a change that requires medical attention. In such an instance, alarm 231 may comprise a vibrator or display or other alarm device to signal the wearer to report to a facility. The alarm may also indicate a point in time when a memory 207 is full of untransmitted images, and the wearer must change their memory card of memory 207 or report to a workstation or other telecommunications facility for image data upload.

In a further embodiment according to FIG. 2, there may be provided a wired remote control for use by a wearer to apply therapeutic ultrasound energy and so control rotation, x and y axis and twist motors 216, 218, 220, 222 and control ultrasonic frequency range of transducer 101, 102, 225 to deliver therapeutic treatment, for example, in the event that a workstation operator is out of wireless contact with the wearer or the wearer has been pre-instructed as to a particular therapeutic treatment that may correct a given complication. A priority of control of an assembly according to FIG.'s 1-4 may be surgeon, primary workstation operator, secondary work station operator and wearer from highest to lowest priority control. Ultrasound waves (as well as OCT and collected infrared) are for the most part harmless. On the other hand, a user may be provided only limited control over, for example, frequency and intensity of any emitted pulsed or continuous wave while a surgeon will have unlimited control especially in emergency intervention situations.

Figure 3 provides an overview of a mechanical arrangement to be contained within a housing 103 of rectangular or square embodiments of a transducer assembly for manipulating a transducer or linear transducer array 225 in two directions, for example, along an x axis 303 and a y axis 304 and to provide an angle of rotation 306 and a twist angle 307 at a desired x, y coordinate pair to redirect a pulsed or continuous sound wave emitted by a transducer or linear or phased array 225 of transducer elements whereby it is envisioned that a footprint on a patient body surface is rectangular or square and relates to the embodiments and circuits of Figures 1 and 2. Assume the rectangle housing 103 comprises guide wires or rods 300, 301, 302 and 303 which may be flexible or body surface conforming on which are provided y-axis rod 304 which may be moved in an up and down direction shown via a corresponding motor 220 and gear assembly not shown to incremental steps along the y axis 304. Similarly, there is provided x-axis rod 305 which may be moved to the left or the right direction shown via corresponding motor 218 and a gear assembly not shown. X-axis rod 305 and Y-axis rod 304 intersect at a desired point where an array or element 225 may be affixed via further rotation and twist motors 216, 222. For example, rotor 306 of motor 216 (in combination with an optional gear assembly 109) provides rotation of a mounted transducer array 102, 225 or transducer element to a predetermined or desired angle of rotation. Motor 222 provides twist 307 to linear or other array 225 or element 102 to change direction of sound wave transmission or reception with π/2 rad (90 degrees) - straight down - being a default position for twist.

Figure 4A provides an exemplary signal content format for providing motor control of a transducer or transducer array 102, 225 of Figures 1, 2 and/or 3 in a direction from a workstation to a remote wired or wireless transducer assembly including a unique transducer transceiver identifier if wireless transmission is utilized. The format also provides for known control such as on/off, focus, depth, mode and the like. According to various embodiments, motor control data may comprise an x direction or a longitudinal axis direction, a perpendicular to x or y direction, an angle of rotation, an angle for twist of a transducer or array 225 as depicted. There is also a DRR field that my be used for all other control mentioned herein including but not limited to: magnification, depth, frequency range, time of day and data (as needed), periodicity of imaging, transducer select data for a selected one from a group or for selecting a group from a larger plurality of transducers, an indicator of a controlling workstation or user or other control indicator that may be included in this field. Other control or other data to be transmitted in a direction from workstation or other user towards an identified transducer assembly may come to mind of one of ordinary skill in the art of ultrasound apparatus. Motor control data may be transmitted, for example, in the form of ultimate desired position or as an incremental step from an actual position or other way that may come to mind of one of skill in the art.

Figure 4B provides an exemplary signal content format for providing a reply signal from a transducer or transducer array of Figure 1, 2 or 3 in a direction from a remote wired or wireless transducer including a unique workstation identifier if wireless transmission is utilized. The wireless transmission may be directed via a server and a server identification provided (for example, of TFM field)) serving one of a plurality of workstations identified by workstation identifier. The format provides for feedback of actual location data of the position of the transducer or transducer array and image data which is preferably compressed. The actual location data may comprise an x axis or longitudinal axis dimension, a perpendicular y axis dimension, an angle of rotation, an angle of twist as well as compressed image data. There may be a TFM indicator field useful for all other control mentioned hereinafter including, for example, the identity of the transducer or transducer array associated with the image data and a time and date indicator provided by the real time clock program of the transducer control unit for the time and date of collection of the image. In an embodiment including a therapeutic transducer, the frequency of operation and the level or magnitude of transmission and a measure of the reflected wave and/or its harmonic may be signaled as well as a data indicator of an image of the region of interest, for example, a blood clot. The actual location data may be compared to a desired location to determine if the remotely manipulatable transducer or transducer array has reached a desired position so that imaging may begin. Moreover, the imaging and control data may be collectively utilized by a workstation to determine the location of the transducer assembly of FIG's. 1, 2 or 3, for example, on the skin surface or within a body cavity.

Figure 5A provides a plurality of suggested locations 502, 503, 504, 505, 507 on a human body for locating a remotely manipulatable ultrasound transducer or transducer array assembly whereby the assemblies may comprise housings and utilize adhesive or elastic material similar to materials used for applying electrodes to a human body as used in electrocardiography. Also, banded adhesive attachments to housings or bandage or elastic banding of the manipulatable transducer or transducer array housing to the human body may be provided. No position on or within a cavity of the human body should be considered to be excluded from reach by a remotely manipulatable transducer or transducer array assembly embodiment. Fixing material for a given position on the patient's body is well known in the field of bandaging from first responder training in medical emergencies and the like.

Image locations 507 or 504 and 502 may be used in combination to provide image locations for the heart and develop three dimensional images thereof in multiple planes. See also FIG. 7, transducers 710, 720, 730. Location 503 may be used to image the liver. Image location 504 may be used to image the carotid artery or neck structure or any gland within the neck. A patient may hold a transducer in their mouth for a period of time in combination with image locations 504 and 505 to image various regions of interest in the neck such as the esophagus. Image location 505 may be used alone or in combination with location 504 to image trans-cranial structures including blood vessels. The fixing material may be a band as in 501 or adhesive tape material as in 2 or affixed via an adhesive as in 503, 504 or 505.

Referring to FIG. 5B, there is shown a single patch 508 having multiple devices for imaging all of the heart, spleen, kidney and liver, individually or in combination. Patch 508 may be worn in the form of an article of clothing such as a vest or worn on a sleeve of a shirt or as an adhesive or semi-permanent plaster, for example, a transducer assembly built into a plaster cast for immobilizing a broken bone. Imaging of the brain or other areas within the skull may be obtained by providing a plurality of transducer assemblies with a single antenna 201 within a skull cap to be worn by a patient. In such a further alternative embodiment, a skull cap may be fashioned to comprise transducer assemblies similar to the plurality shown as patch 508 (FIG. 5B) sharing a single antenna 201, transceiver 205 and memory 207. The skull cap may be worn and comprise a plurality of remotely controlled wireless or wired ultrasonic transducer assemblies for imaging interior portions of the skull with x, y, rotation and twist motors per Figures 2-3. A longitudinal cylindrically shaped transducer may be positioned in the rectal or vaginal cavity for x, rotation and twist to capture plural views, for example, of the prostrate (rectal) or a fetus (vaginal). Vaginal sonography may be used for evaluation of non-palpable masses, uterine and cervical lesions and the like. In one embodiment of a vaginal wireless transducer assembly, the assembly may comprise an intrauterine device (IUD) for birth control purposes.

Now, some specific applications of the remotely manipulatable ultrasonic transducer apparatus of Figures 1-5 will now be discussed with reference to Figures 6 and 7. Figure 6 represents a fetus imaging and Figure 7 an imaging during a heart operation.

### Fetus monitor

Figure 6 provides an exemplary application of a manipulatable transducer array that may be worn by a pregnant female in the abdominal area for remotely monitoring a fetus for, for example, extraordinary conditions. Let us assume that a female patient has been checked into a maternity ward of a hospital. A rectangular, remotely manipulatable transducer array assembly 610 shaped in the form of a curvilinear or convex abdominal transducer 225 mounted to appropriately shaped rod apparatus per FIG. 3 may be affixed as shown in Figure 6 by adhesive to the abdominal cavity and appropriately be provided with a surface that is sufficiently curved as to maintain contact with a, for example, 10 cm by 12 centimeter rectangular skin surface region of interest. For example, such surface of the housing 103 may be made of a giving material such as rubber and the transducer assembly contained within the housing at sufficient depth to permit the curved skin surface to be absorbed within a curved surface of array assembly 610 and maintain constant skin contact. The rods 301-305 comprising the assembly of Figure 3 may be flexible or fixed to conform to the surface on which the assembly of Figure 3 is mounted. Thus, the assembly may be movable, yet, semi-rigid and the mounted curvilinear or convex transducer or transducer array adapted to move over as large an area as possible, perhaps mounted in an article of clothing such as a girdle. The patient typically is prone to considerable movement during pre-birth and requires considerable attention. The housing must be firmly secured by adhesive material. Fetus monitoring equipment may be used in concert with the transducer array assembly 610 and the fetus remotely monitored for activity such as turning or stage of birth canal entry and passage via the remotely manipulatable transducer or transducer array assembly 610 shown. Typically, bed-side space is at a premium as the father or other coach and nurses are present at bedside. Also, periodically, a physician or surgeon will want to check the degree of opening of the birth canal for delivery. A rectangular housing 610 affixed (or worn as an article of clothing) as shown in Figure 6 permits remote monitoring of a fetus during pre-birth and birth from a remote workstation, saving valuable bed-side space and providing an invaluable supplement to normal fetus monitoring devices, for example, for monitoring fetus heart rate and pressure. Moreover, a second array assembly 610 may be affixed to the patient's back or side or worn as part of the same article of clothing to provide a further view of the fetus from the opposite side. For three dimensional imaging, it is preferable that the first and second assemblies 610 not be coaxial - directed at one another. Each assembly may be provided as discussed above with transducer array assemblies operating at different frequency ranges to permit both therapeutic and imaging functionality to occur simultaneously. Moreover, one assembly 610 may be fixed to the skin surface to provide remotely manipulatable therapeutic use and another provided for imaging purposes.

Assume also a scenario where the pregnancy may have complications and an outpatient pregnant woman wears one or more assemblies 610 in accordance with FIG.'s 1-4 and FIG. 6. The pregnant woman may transmit image data periodically to a remote workstation. The workstation attendant may, responsive to receipt of image data, signal a complication to the pregnant woman by actuating alarm 231. In response, the pregnant woman may contact the workstation attendant and be directed to immediately report to a physician for assistance.

### Minimally-invasive pericardial surgery

Figure 7 shows an arrangement for pericardial activity and possibly surgery including first and second remotely manipulatable transducer assemblies, 710, 720 mounted to either side of, for example, an image guided catheter 730 surgical location as one example of an application for minimally invasive heart surgery. The image guided catheter 730 may be one as shown and described in my co-pending United States Patent Application Serial No. 11/782,991, filed July 25, 2007, entitled "Image Guided Catheters and Methods of Use," of the same inventor, for example, per Figure 1 of that application, my published PCT application WO2008/046031, United States Patent Application Serial No.'s 11/871,219 and 11/871,282 filed October 12, 2007. Methods of use include, for example, double balloon heart wall processes, cavity drainage and the like wherein the wireless arrays 710, 730 may image successful deployment of the image guided catheter and intervention, and therapeutic or diagnostic apparatus in the region of interest of the heart. Elements or transducer arrays 710, 720, 730 may cooperate to provide stereoscopic as well as multi-planar imaging while the image-guided catheter 730 contains its own ultrasonic array for imaging a point of intervention, therapeutic or diagnostic care. A single workstation may receive imaging and control signal data as described in the context of FIG. 4B from each of transducer array assemblies 710, 720 and 730.

A workstation for use with such an arrangement shown in Figure 7 may comprise a plurality of screens to show all images obtained from each of ultrasound transducer elements or arrays 710, 720 and 730. An operator remote from the operating suite may, for example, remotely manipulate and control arrays 710, 720 and obtain imaging therefrom while the surgeon may receive imaging data from imaging catheter 730 on a surgical room display. The workstation operator may communicate with a surgeon by a wireless telecommunications device worn, for example, as a headset by each of the surgeon (or other operating room personnel) and the workstation operator. The transducers/array assemblies 710, 720 including motors may be remotely manipulated on the patient within a region of interest from the workstation and remain on the patient during the entire operation. The imaging can occur when and as requested by the surgeon of the workstation operator. Also, transducers/array assemblies 710, 720 may comprise transducers for therapeutic purposes, for example, for dissolving a blood clot and so be controlled by the surgeon or the workstation operator for that purpose (with the surgeon having the ultimate control). Uses may comprise, for example, but not be limited to pericardiocentesis, vascular surgery, coronary angioplasty, valvuloplasty, alcohol septal ablation, delivery of drugs, stem cells or laser therapy, valve repair or replacement, cardiac shape modifying devices such as ACORN-like or MYOSPLINT™, myocardial scar reconstruction, ventricular reconstruction and ventricular assist device placement. One may monitor the blood flow of any vessel to and from the heart or carotid blood flow during cranial or other surgery. Besides the image guided catheter 730, other devices usable with a remotely manipulatable transducer or transducer array assembly 710, 720 include a biopsy forceps, a drainage catheter, a pressure monitoring system, a suture application system, a therapy delivery catheter or system or other intervention system.

Figure 8A shows a workflow scenario whereby the primary workstation is remote from a A. fluoroscopy, B. operating room and the like and, if necessary, shielded from adverse impacts of radiation such as magnetic resonance. One further limitation of using ultrasound in an operating suite is electrocautery procedures which may degrade collected image signal quality when the electrocautery apparatus is in use. Referring to Figure 8A, there may be situations where the ultrasound operator at primary workstation be optimally protected, for example, in a fluoroscopy suite where the operator is exposed to radiation and would otherwise need a heavy lead suit. Even with the lead suit, the operator would typically have to move a 400 or 500 pound ultrasound machine back and forth from along side the operating table to away from the table when a C arm is being used. Now, the ultrasound operator may sit at a remote primary workstation after the operator or the surgeon placing the remotely manipulatable transducer or transducer array assembly 225 on the surgical patient and then sit behind a lead shield to manipulate and operate the transducer element or transducer array remotely at their primary workstation. Note that a plurality of displays are provided at the primary workstation as depicted for providing multiple views, stereoscopic views and the like obtainable from all three transducers or array asseblies 710, 720, 730 of FIG. 7 or other transducers not shown. Also, there is typically inadequate space in an operating room for an ultrasound operator, for example, next to a surgical operating table. The operator or surgeon may place the transducer assemblies 710, 720 and then remotely manipulate and control and view images from the remotely manipulatable transducer assembly or apply therapeutic treatment. Their workstation can be located in a corner of the operating room or outside the operating room and the operator communicate by telecommunications means with the surgeon (or other operating room personnel).

Also shown in Figure 8A is a remote workstation which may be a back-up for primary workstation and communicate in a wireless or by wired (network cable) to primary workstation. The remote workstation may be in telecommunications contact with primary workstation and with the surgeon of a fluoroscopy suite A or an operating room B. Primary and remote workstations may communicate with a server for coordinating field emergency service C. If the primary workstation is dealing with an ongoing operation, a medical field emergency may occur. An emergency medical technician at field emergency C may be guided to apply one or more remotely manipulatable transducer assemblies to an emergency patient and that patient be provided with therapeutic or imaging from a primary or remote workstation. A plurality of transducer assemblies may be placed (or worn) as per Figure 5B in areas of trauma such as the heart, liver and kidney and images transmitted to an emergency room to which the victim is being transported. Coordination between an ambulance and an emergency room may be improved by remote imaging and to plan for victim arrival. In a field emergency C or battlefield situation, remote imaging by a transducer assembly of Figures 1-5 may assist in triage decisions such as where to transport a patient. In another application, patients require constant monitoring in an intensive care unit and a plurality of assemblies per FIG. 5B may be utilized to monitor a plurality of sites remotely involved in a surgery. In an alternative embodiment, the assemblies may be embedded within a surgical dressing. Thus ultrasound medical images (OCT, infrared or other medical imaging) may be obtained without having to remove dressings and break a sterile field.

FIG. 8B provides details of wired/wireless connections between a patient, an image generator and a remote terminal, for example, in the scenario of FIG. 8A. In particular, a patient is shown wearing a transducer assembly 507 for imaging, for example, the kidney. The transducer assembly 507 may comprise a surgical dressing, a girdle or be fixed to the skin surface with adhesive. The assembly 507 may comprise an antenna 201 or be connected by a cable 104 in a wireless or wired manner respectively to a patient-side or closely located (such as in a shielded location) image generator and display 801. The assembly 507 may be directly connected by wired or wireless connection to a remote terminal 802 or connected via local image generator and display 801. Moreover, any of elements 507, 801 and 802 may comprise a controller for running software such as *pcAnywhere* software for secure intranet or internet connection. Similarly, a wireless connection may provide similar features of data compression and data encryption as provided by known software or a known hardware data compressor 212.

Figure 9 shows another workflow scenario for describing, for example, remote field emergency use, operating room B, fluoroscopy suite A use and primary and alternative lead shielded primary workstations connected by a wireless or wired network such as a telecommunications network. Regardless of the patient location, even if the patient is considered a field emergency, the remotely manipulatable transducer assembly may be placed by an emergency first responder, remotely manipulated to a desired location on the patient body surface and image data remotely transmitted to a remote workstation. The emergency first responder can respond to simple commands to appropriately place or relocate one or more transducers on the victim, for example, via a telecommunications channel. For example, first responder personnel at a remote location in Alaska where there is a medical emergency can be remotely guided by a hospital in Juneau to properly place a remotely manipulatable transducer or transducer array assembly at a location on the body surface, and the image data may be conveyed by satellite telecommunications to Juneau as control data is transmitted on the reverse path from a remote operator/manipulator. A second communications channel may used by the operator to guide the first responder as to the proper placement of the one or more remotely manipulatable transducer or transducer array assemblies. Thereafter, the remote operator in Juneau may move the transducer array remotely and control the diagnostic, therapeutic or interventional imaging. The embodiments described above may extend telemedicine and health care to remote areas and underdeveloped countries. Semi-skilled health workers can place one or more transducer assemblies on a patient or victim in such an underdeveloped geographic area and a specialist perform a remote diagnosis and treatment from a hospital or office thousands of miles away.

### Exemplary Application

A prototype transducer assembly similar to that depicted in FIG. 1 has been constructed comprising a 7 MHz, phased array, multi-frequency, broadband transducer. The transducer phased array 102 provides a 1 cm footprint and the overall size of the housing 103 is 1.5 cm including motor and control circuits. The rotation motor is capable of rotating the phased transducer array 0 to π rad (0-180°) in plane. An ultrasound coupling gel is applied to the region of fixation. Fixation was achieved using a stretchable adhesive tape to the skin surface of a patient above the liver (Figure 10), the heart (Figure 11) and the abdominal aorta (Figure 12). The devices were fixed, and the ultrasound workstation is operated remotely from the patient via a lengthy control cable 104. The workstation operator remotely controlled a rotation motor 216 of the housing 103. The workstation was provided with application software for remote control and collection of image data. The remote workstation operator remotely controlled the transducer and rotation motor using conventional and special workstation input apparatus to select rotation angle, control (adjust) the depth of field, image brightness, contrast, focal length, sweep speed, to select color Doppler three dimensional imaging (Figure 10(c)) from conventional gray scale using the single rotating transducer and to measure Doppler velocities. The image data was recorded as conventional JPEG or AVI format in memory for transfer to an appropriate recording medium (such as magnetic tape or DVD).

Figure 10 provides views of a liver of a patient obtained remotely from a patient via an assembly embodiment constructed similarly to that depicted in Figure 1 and affixed to a skin surface by stretchable adhesive tape. Figure 10(a) represents an image at 0 rad rotation, Figure 10(b) represents an image at π/3 rad (60°) rotation as examples of image planes. Figure 10(c) represents a three dimensional color Doppler image shown in gray scale obtained by use of the single rotatable transducer affixed to skin surface proximate to the liver. Figure 11 provides views of a heart obtained via an assembly embodiment constructed similarly to that depicted in Figure 1 wherein Figure 11 (a) represents an image taken at 0rad rotation and Figure 11(b) represents an image taken at π/3 rad (60°) rotation. In FIG. 11 (a) and FIG. 11(b), the left ventricle, the mitral valve and the left atrium of the heart have been located and are identified Figure 12 provides a view of the abdominal aorta obtained via an assembly embodiment constructed similarly to that depicted in Figure 1. An arrow is used to locate the abdominal aorta in the image. All images were obtained by fixing the device and without any operator intervention at patient- side after fixing the assembly at a given location.

The principles of application of the several discussed embodiments of a remotely manipulatable medical imager may be extended to other embodiments such as infrared temperature imaging and optical coherence tomography imaging so long as the assembly for remotely manipulating and capturing images may be sufficiently small in size to permit comfortable attachment or wearing on or within the body. For example, any active or passive medical imaging device for remote manipulation should be as small as possible just as circuitry and motors for control, recording and transmission of imaging may be made as small in size as possible. These and other features of embodiments and aspects of a remotely manipulatable ultrasound transducer or transducer array assembly may come to mind from reading the above detailed description and any claimed invention should be only deemed limited by the scope of the claims to follow.

## Claims

1. A remotely manipulatable ultrasound transducer assembly for communicating with a remote workstation including a display wherein said assembly comprises at least one linear transducer array (102) for collecting ultrasound images and a first motor (216) for rotating the linear transducer array (102), wherein
the first motor (216) is adapted for rotating said transducer array (102) incrementally through an angle less than or equal to π rad (180 degrees) in a plane parallel to an external surface of an animal body when said transducer assembly is fixed to the external surface of the animal body and a plurality of transducer elements forming the linear array, are located on a diameter of a circular cross section for rotation with respect to the external surface of the animal body; the ultrasound transducer assembly further comprises
a second motor (222) for twisting the linear transducer array located on the diameter;
a housing (103) for the first and second motors (216, 222) the linear transducer array (102), the circular cross section and the diameter for mounting said linear transducer array for rotation and twist by said first rotation and second twist motors (216, 222), said housing (103) having a surface for fixation to said external animal body surface by fixing material (105), said linear transducer array (102) being adapted for rotation by said first motor (216) in the plane parallel to the external animal body surface and being mounted on the diameter of the circular cross section for being twisted about an axis formed by the diameter of the circular cross section,
the housing further comprising
a pair of perpendicular rods (300, 301) to which said linear transducer array is located at their juncture,
a third x axis motor (218) and a fourth y axis motor (220) for moving said linear transducer array via the pair of perpendicular rods in the plane parallel to the external body surface to a desired location for imaging internal animal body parts,
a wireless transceiver (205) for communicating with a remote workstation; and
a controller (210) for receiving motor control data from said transceiver (205) and controlling said first motor (216) for rotation, said second motor (222) for twist, said third x axis motor (218) and said fourth y axis (220) motor responsive to receipt of said motor control data to relocate the linear transducer array (102) to a different imaging location on the animal body surface.

2. The remotely manipulatable ultrasound transducer assembly as recited in claim 1, further **characterized by** the second motor for twisting said linear transducer array from a default position, said linear transducer array directing sound waves into an animal body from the surface to which it is fixable, to another rotational angular position in response to actuation of said first motor for rotation, said linear transducer array for further directing sound waves from said body surface into said body for imaging according to one of an input incremental and final angle of direction of twist responsive to actuation of said second motor for twist.

3. The remotely manipulatable ultrasound transducer assembly as recited in claims 2 or 1 **further characterized by** the third x axis motor and the fourth y axis motor for moving said linear transducer array of said housing in two directions within a two-dimensional footprint of movement of said housing when the assembly is fixed on the animal body surface.

4. The remotely manipulatable ultrasound transducer assembly as recited in one of claims 1 or 2, wherein said motor for rotating is **further characterized by** a gear assembly coupled between said rotation motor and said linear transducer array for incrementally rotating said linear transducer array through an angle of rotation up to π rad (180 degrees) to collect image data of a plurality of image planes.

5. The remotely manipulatable ultrasound transducer assembly as recited in one of claim 1 or 2for cooperating with a second such assembly for fixation to a second different position on the external body surface **further characterized by** a capability of the first and second assembly cooperating to provide three-dimensional, stereoscopic imaging of a region of interest of said animal body under control of respective controllers and responsive to control signals received by respective wireless transceivers.

6. The remotely manipulatable ultrasound transducer assembly of one of claim 1 or 2, wherein said first motor for rotation is **further characterized by** an associated gear assembly for accurate rotational movement of said linear array of transducer elements of said diameter in response to remote wireless control signals received at said wireless transceiver.

7. The remotely manipulatable ultrasound transducer assembly as recited in one of claims 1 or 2 , **further characterized by:**
a memory for temporary storage of image data captured from rotation of said linear transducer array for transmission via said wireless transceiver.

8. The remotely manipulatable ultrasound transducer assembly as recited in claims 1 or 2 **further characterized by:**
an antenna, coupled to said wireless transceiver, for receiving wireless radio signals from said remote workstation and for transmitting wireless radio signals to said remote workstation.

9. The remotely manipulatable ultrasound transducer assembly as recited in claims 1 or 2, **further characterized by:**
a triggerable alarm for signaling a user of a predetermined condition responsive to receipt of an actuation signal from a remote site via said wireless transceiver.

10. The remotely manipulatable ultrasound transducer assembly as recited in claim 2, **further characterized by:**
a controller for controlling said rotation motor according to remote control input;
a battery power supply for powering elements of the transducer assembly requiring power and;
a memory for temporary storage of image data captured from rotation of said linear transducer array.

11. The remotely manipulatable ultrasound transducer assembly as recited in claims 1 or 2, **further characterized by**
said wireless transceiver reporting actual position data of one of said linear transducer array to a remote workstation comprising rotation, twist and x and y locations,
wherein said actual position data comprises values including linear transducer array on/off, focus, depth, angle of rotation and angle of twist.

12. The remotely manipulatable ultrasound transducer assembly as recited in claim 7 **further characterized by:**
an output of said linear transducer array being provided to an analog to digital convertor for sampling prior to storage in said memory.

13. The remotely manipulatable ultrasound transducer assembly as recited in one of claim 12 **further characterized in that**
said output of said linear transducer array is compressed at a data compressor responsive to said analog to digital converter prior to said transmission via said wireless transceiver.

14. The remotely manipulatable ultrasound transducer assembly as recited in claim 1 or 2, **further characterized by:**
a removable memory coupled to said controller for storing and uploading image data to a workstation.

15. The remotely manipulatable ultrasound transducer assembly as recited in one of claims 1 or 2 , wherein said linear transducer array is **further characterized by:**
a unique identification code for uniquely identifying said transducer array to a remote workstation.

16. The remotely manipulatable ultrasound transducer assembly as recited in claim 7 **further characterized in that**
image data wirelessly transmitted from said controller memory via said wireless transceiver to a remote workstation is subsequently retransmitted to and received at said wireless transceiver of said assembly for verification with said wirelessly transmitted image data stored in said controller memory.

17. The remotely manipulatable ultrasound transducer assembly as recited in one of claims 1 or 2, **further characterized by**
an input for controlling the linear transducer array to deliver therapeutic ultrasound waves to a specified region of the animal body, said input further capable of changing one of a rotation and a twist angle of propagation by said linear transducer array.

18. The remotely manipulatable ultrasound transducer assembly as recited in one of claims 1 or 2, **characterized by** the first linear transducer array operating at a first frequency range for therapeutic treatment and at a second frequency range higher than said first frequency range for imaging.

19. The remotely manipulatable ultrasound transducer assembly as recited in claims 1 or 2 , wherein said assembly is **further characterized by:**
a rod construction in the form of a rectangle comprising two pairs of parallel rods forming the rectangle,
each parallel pair of rods conducting a further perpendicular rod member in one of x axis and y axis directions, the transducer array for connection to the juncture of the two perpendicular rod members, the two perpendicular rod members for movement of said transducer array as determined by the third x axis and fourth y axis motors and in response to control data received at said wireless transceiver.

20. The remotely manipulatable ultrasound transducer assembly as recited in claim 19 wherein said rods are flexible and in combination are adaptable for conforming to a non-flat animal surface area.

21. The remotely manipulatable ultrasound transducer assembly as recited in claims 1or 2 , **further characterized by:**
a wired remote control input to the controller of the assembly for use by a human user of the assembly in controlling assembly operation,
the wired remote control input adapted to control an output of sound waves in a therapeutic manner to a region of interest by inputting an angle of twist, an angle of rotation and a depth of transmission via said wired remote control.

22. The remotely manipulatable ultrasound transducer assembly as recited one of claims 1 or 2, **further characterized by** cooperation with an image guided catheter for imaging a region of interest of said animal body, the image guided catheter for use in minimally invasive surgical procedures for one of therapeutic, diagnostic and interventional purposes at said region of interest.

## Patentansprüche

1. Fernbedienbare Ultraschallwandlerbaugruppe zum Kommunizieren mit einer entfernten Arbeitsstation, die ein Display beinhaltet, wobei die Baugruppe wenigstens ein lineares Wandler-Array (102) zum Sammeln von Ultraschallbildern und einen ersten Motor (216) zum Drehen des linearen Wandler-Arrays (102) umfasst, wobei
der erste Motor (216) zum inkrementalen Drehen des Wandler-Arrays (102) um einen Winkel von gleich oder weniger als π rad (180 Grad) in einer Ebene parallel zu einer Außenfläche eines Tierkörpers ausgelegt ist, wenn die Wandlerbaugruppe an der Außenfläche des Tierkörpers befestigt ist, und mehrere das Linear-Array bildende Wandlerelemente sich in einem Durchmesser eines kreisförmigen Querschnitts zum Rotieren mit Bezug auf die Außenfläche des Tierkörpers befinden; wobei die Ultraschallwandlerbaugruppe ferner Folgendes umfasst:
einen zweiten Motor (222) zum Verdrehen des auf dem Durchmesser befindlichen linearen Wandler-Arrays;
ein Gehäuse (103) für den ersten und zweiten Motor (216, 222), das lineare Wandler-Array (102), den kreisförmigen Querschnitt und den Durchmesser zum Montieren des linearen Wandler-Arrays zwecks Rotation und Verdrehung durch den ersten Rotations- und den zweiten Verdrehmotor (216, 222), wobei das Gehäuse (103) eine Oberfläche zum Befestigen an der Außenfläche des Tierkörpers mit Befestigungsmaterial (105) aufweist, wobei das lineare Wandler-Array (102) für eine Rotation durch den ersten Motor (216) in der Ebene parallel zur Außenfläche des Tierkörpers ausgelegt und auf dem Durchmesser des kreisförmigen Querschnitts montiert ist, um um eine vom Durchmesser des kreisförmigen Querschnitts gebildete Achse verdreht zu werden,
wobei das Gehäuse ferner ein Paar lotrechte Stäbe (300, 301) umfasst, an deren Verbindungsstelle sich das lineare Wandler-Array befindet,
einen dritten x-Achsen-Motor (218) und einen vierten y-Achsen-Motor (220) zum Bewegen des linearen Wandler-Arrays über das Paar lotrechte Stäbe in der Ebene parallel zur Außenfläche des Körpers zu einer gewählten Stelle zum Abbilden von Innenteilen des Tierkörpers,
einen drahtlosen Transceiver (205) zum Kommunizieren mit einer entfernten Arbeitsstation; und
eine Steuerung (210) zum Empfangen von Motorsteuerdaten von dem Transceiver (205) und zum Steuern des ersten Motors (216) zur Rotation, des zweiten Motors (222) zum Verdrehen, des dritten x-Achsen-Motors (218) und des vierten y-Achsen-Motors (220) als Reaktion auf den Empfang der Motorsteuerdaten zum Verlegen des linearen Wandler-Arrays (102) zu einem anderen Abbildungsort auf der Oberfläche des Tierkörpers.

2. Fernbedienbare Ultraschallwandlerbaugruppe nach Anspruch 1, ferner **gekennzeichnet durch** den zweiten Motor zum Verdrehen des linearen Wandler-Arrays von einer Vorgabeposition, wobei das lineare Wandler-Array Schallwellen von der Oberfläche, an der er befestigt werden kann, in einen Tierkörper leitet, zu einer anderen Drehwinkelposition als Reaktion auf das Betreiben des ersten Motors zur Rotation, wobei das lineare Wandler-Array ferner Schallwellen von der Körperoberfläche in den Körper zur Bilderzeugung gemäß einem von einem Eingangsinkrement oder einem Verdrehrichtungsendwinkel als Reaktion auf das Betreiben des zweiten Verdrehmotors leitet.

3. Fernbedienbare Ultraschallwandlerbaugruppe nach Anspruch 2 oder 1, ferner **gekennzeichnet durch** den dritten x-Achsen-Motor und den vierten y-Achsen-Motor zum Bewegen des linearen Wandler-Arrays des Gehäuses in zwei Richtungen innerhalb eines zweidimensionalen Bewegungsprofils des Gehäuses, wenn die Baugruppe an der Oberfläche des Tierkörpers befestigt ist.

4. Fernbedienbare Ultraschallwandlerbaugruppe nach Anspruch 1 oder 2, wobei der Motor zur Rotation ferner **gekennzeichnet ist durch** eine Getriebebaugruppe, die zwischen dem Rotationsmotor und dem linearen Wandler-Array gekoppelt ist, zum inkrementalen Drehen des linearen Wandler-Arrays um einen Drehwinkel von bis zu π rad (180 Grad), um Bilddaten von mehreren Bildebenen zu sammeln.

5. Fernbedienbare Ultraschallwandlerbaugruppe nach Anspruch 1 oder 2, zum Zusammenwirken mit einer zweiten solchen Baugruppe zum Befestigen in einer zweiten anderen Position an der Außenfläche des Körpers, ferner **gekennzeichnet durch** eine Fähigkeit der ersten und zweiten Baugruppe, die zusammenwirken, um eine dreidimensionale stereoskopische Abbildung einer Region von Interesse des Tierkörpers unter der Steuerung von jeweiligen Steuerungen und als Reaktion auf von jeweiligen drahtlosen Transceivern empfangenen Steuersignalen bereitzustellen.

6. Fernbedienbare Ultraschallwandlerbaugruppe nach Anspruch 1 oder 2, wobei der erste Motor zur Rotation ferner **gekennzeichnet ist durch** eine assoziierte Getriebebaugruppe für eine genaue Drehbewegung des linearen Arrays von Wandlerelementen mit dem Durchmesser als Reaktion auf an dem drahtlosen Transceiver empfangene drahtlose Fernsteuersignale.

7. Fernbedienbare Ultraschallwandlerbaugruppe nach Anspruch 1 oder 2, die ferner **gekennzeichnet ist durch**:
einen Speicher zum vorübergehenden Speichern von von der Rotation des linearen Wandler-Arrays erfassten Bilddaten zur Übertragung über den drahtlosen Tansceiver.

8. Fernbedienbare Ultraschallwandlerbaugruppe nach Anspruch 1 oder 2, ferner **gekennzeichnet durch**:
eine mit dem drahtlosen Transceiver gekoppelte Antenne zum Empfangen von drahtlosen Funksignalen von der entfernten Arbeitsstation und zum Übertragen von drahtlosen Funksignalen zu der entfernten Arbeitsstation.

9. Fernbedienbare Ultraschallwandlerbaugruppe nach Anspruch 1 oder 2, ferner **gekennzeichnet durch**:
einen auslösbaren Alarm, um einem Benutzer eine vorbestimmte Bedingung als Reaktion auf den Empfang eines Betätigungssignals von einem entfernten Ort über den drahtlosen Transceiver zu signalisieren.

10. Fernbedienbare Ultraschallwandlerbaugruppe nach Anspruch 2, ferner **gekennzeichnet durch**:
eine Steuerung zum Steuern des Rotationsmotors gemäß Fernsteuereingaben;
eine Batteriestromversorgung zum Speisen von Elementen der Strom benötigenden Wandlerbaugruppe, und
einen Speicher zum vorübergehenden Speichern von bei der Rotation des linearen Wandler-Arrays erfassten Bilddaten.

11. Fernbedienbare Ultraschallwandlerbaugruppe nach Anspruch 1 oder 2, ferner **dadurch gekennzeichnet, dass**
der drahtlose Transceiver Ist-Positionsdaten von einem aus dem linearen Wandler-Array einer entfernten Arbeitsstation meldet, umfassend Rotation, Verdrehung sowie x-und y-Orte,
wobei die Ist-Positionsdaten Werte einschließlich linearer Wandler-Array ein/aus, Fokus, Tiefe, Rotationswinkel und Verdrehwinkel umfasst.

12. Fernbedienbare Ultraschallwandlerbaugruppe nach Anspruch 7, die ferner **dadurch gekennzeichnet ist, dass**
ein Ausgang des linearen Wandler-Arrays an einen Analog-Digital-Wandler zum Abtasten vor der Speicherung in dem Speicher angelegt wird.

13. Fernbedienbare Ultraschallwandlerbaugruppe nach Anspruch 12, die ferner **dadurch gekennzeichnet ist, dass**
der Ausgang des linearen Wandler-Arrays an einem Datenkompressor als Reaktion auf den Analog-Digital-Wandler vor der Übertragung über den drahtlosen Transceiver komprimiert wird.

14. Fernbedienbare Ultraschallwandlerbaugruppe nach Anspruch 1 oder 2, ferner **gekennzeichnet durch**:
einen mit der Steuerung gekoppelten entfernbaren Speicher zum Speichern und Heraufladen von Bilddaten zu einer Arbeitsstation.

15. Fernbedienbare Ultraschallwandlerbaugruppe nach Anspruch 1 oder 2, wobei das lineare Wandler-Array ferner **gekennzeichnet ist durch**:
einen eindeutigen Identifikationscode, um das Wandler-Array einer entfernten Arbeitsstation eindeutig zu identifizieren.

16. Fernbedienbare Ultraschallwandlerbaugruppe nach Anspruch 7, die ferner **dadurch gekennzeichnet ist, dass**
Bilddaten, die drahtlos vom Speicher der Steuerung über den drahtlosen Transceiver zu einer entfernten Arbeitsstation übertragen werden, nachfolgend erneut zum drahtlosen Transceiver der Baugruppe übertragen und von diesem empfangen werden, zur Überprüfung mit den im Speicher der Steuerung gespeicherten drahtlos übertragenen Bilddaten.

17. Fernbedienbare Ultraschallwandlerbaugruppe nach Anspruch 1 oder 2, ferner **gekennzeichnet durch**
einen Eingang zum Steuern des linearen Wandler-Arrays zum Zuführen von therapeutischen Ultraschallwellen zu einer bestimmten Region des Tierkörpers, wobei der Eingang ferner eines von einem Rotations- oder einem Ausbreitungsverdrehwinkel **durch** das lineare Wandler-Array ändern kann.

18. Fernbedienbare Ultraschallwandlerbaugruppe nach Anspruch 1 oder 2, **dadurch gekennzeichnet, dass** das erste lineare Wandler-Array mit einem ersten Frequenzbereich zur therapeutischen Behandlung und mit einem zweiten Frequenzbereich zur Bilderzeugung arbeitet, der höher ist als der erste Frequenzbereich.

19. Fernbedienbare Ultraschallwandlerbaugruppe nach Anspruch 1 oder 2, wobei die Baugruppe ferner **gekennzeichnet ist durch**:
eine Stabkonstruktion in Form eines Rechtecks, die zwei Paare von parallelen das Rechteck bildenden Stäben umfasst,
wobei jedes parallele Paar Stäbe ein weiteres lotrechtes Stabelement in einer von der x-Achsen- und der y-Achsen-Richtung, das Wandler-Array zur Verbindung mit der Verbindungsstelle der beiden lotrechten Stabelemente, die beiden lotrechten Stabelemente zum Bewegen des Wandler-Arrays, wie **durch** den dritten x-Achsen- und den vierten y-Achsen-Motor festgelegt, und als Reaktion auf am drahtlosen Transceiver empfangene Steuerdaten leitet.

20. Fernbedienbare Ultraschallwandlerbaugruppe nach Anspruch 19, wobei die Stäbe flexibel sind und in Kombination an einen nicht flachen Oberflächenbereich des Tiers angepasst werden können.

21. Fernbedienbare Ultraschallwandlerbaugruppe nach Anspruch 1 oder 2, ferner **gekennzeichnet durch**:
einen verdrahteten Fernsteuereingang in die Steuerung der Baugruppe zur Verwendung **durch** einen menschlichen Benutzer der Baugruppe beim Steuern des Betriebs der Baugruppe,
wobei der verdrahtete Fernsteuereingang zum Steuern eines Ausgangs von Schallwellen auf therapeutische Weise in eine Region von Interesse **durch** Eingeben eines Verdrehwinkels, eines Rotationswinkels und einer Übertragungstiefe über die verdrahtete Fernsteuerung ausgelegt ist.

22. Fernbedienbare Ultraschallwandlerbaugruppe nach Anspruch 1 oder 2, ferner **gekennzeichnet durch** eine Zusammenwirkung mit einem bildgeführten Katheter zum Abbilden einer Region von Interesse des Tierkörpers, wobei der bildgeführte Katheter zur Verwendung in minimal-invasiven chirurgischen Prozeduren für eines von einem therapeutischen, diagnostischen oder interventionellen Zweck an der Region von Interesse dient.

## Revendications

1. Ensemble formant transducteur à ultrasons pouvant être manipulé à distance pour communiquer avec un poste de travail à distance comprenant un affichage, dans lequel ledit ensemble comprend au moins un réseau de transducteurs linéaire (102) permettant de collecter des images ultrasonores et un premier moteur (216) permettant de faire tourner le réseau de transducteurs linéaire (102), dans lequel
le premier moteur (216) est adapté pour faire tourner ledit réseau de transducteurs (102) de manière incrémentielle d'un angle inférieur où égal π rad (180 degrés) dans un plan parallèle à une surface externe d'un corps d'animal lorsque ledit ensemble formant transducteur est fixé à la surface externe du corps d'animal, et une pluralité d'éléments de transducteurs formant le réseau linéaire sont situés sur un diamètre d'une section transversale circulaire en vue d'une rotation par rapport à la surface externe du corps d'animal ; l'ensemble formant transducteur à ultrasons comprend en outre
un second moteur (222) permettant une déformation du réseau de transducteurs linéaire situé sur le diamètre ;
un boîtier (103) pour les premier et second moteurs (216, 222), le réseau de transducteurs linéaire (102), la section transversale circulaire et le diamètre permettant un montage dudit réseau de transducteurs linéaire en vue d'une rotation et d'une déformation grâce auxdits premier moteur de rotation et second moteur de déformation (216, 222), ledit boîtier (103) présentant une surface en vue d'une fixation à ladite surface externe de corps d'animal grâce à un matériau de fixation (105), ledit réseau de transducteurs linéaire (102) étant adapté pour tourner grâce audit moteur (216) dans le plan parallèle à la surface externe de corps d'animal et étant monté sur le diamètre de la section transversale circulaire de manière à être déformé autour d'un axe formé par le diamètre de la section transversale circulaire,
le boîtier comprenant en outre
une paire de tiges perpendiculaires (300, 301) au niveau de la jonction desquelles est situé ledit réseau de transducteurs linéaire,
un troisième moteur d'axe x (218) et un quatrième moteur d'axe y (220) permettant de déplacer ledit réseau de transducteurs linéaire par l'intermédiaire de la paire de tiges perpendiculaires dans le plan parallèle à la surface externe de corps jusqu'à un emplacement souhaité en vue d'une imagerie de parties internes du corps d'animal,
un émetteur-récepteur sans fil (205) permettant de communiquer avec un poste de travail à distance ; et
un dispositif de commande (210) permettant de recevoir des données de commande de moteur en provenance dudit émetteur-récepteur (205) et de commander ledit premier moteur (216) permettant une rotation, ledit second moteur (222) permettant une déformation, ledit troisième moteur d'axe x (218) et ledit quatrième moteur d'axe y (220) en réaction à une réception desdites données de commande de moteur afin de resituer le réseau de transducteurs linéaire (102) à un emplacement d'imagerie différent sur la surface de corps d'animal.

2. Ensemble formant transducteur à ultrasons pouvant être manipulé à distance selon la revendication 1, **caractérisé en outre par** le second moteur permettant de déformer ledit réseau de transducteurs linéaire à partir d'une position par défaut, ledit réseau de transducteurs linéaire dirigeant des ondes sonores jusque dans un corps d'animal à partir de la surface sur laquelle il peut être fixé, vers une autre position angulaire rotationnelle en réaction à l'activation dudit premier moteur permettant une rotation, ledit réseau de transducteurs linéaire permettant en outre de diriger des ondes sonores à partir de ladite surface de corps jusque dans ledit corps en vue d'une imagerie selon un angle parmi un angle incrémentiel d'entrée et un angle final de direction de déformation en réaction à une activation dudit second moteur permettant une déformation.

3. Ensemble formant transducteur à ultrasons pouvant être manipulé à distance selon la revendication 2 ou 1, **caractérisé en outre par** le troisième moteur d'axe x et le quatrième moteur d'axe y permettant de déplacer ledit réseau de transducteurs linéaire dudit boîtier dans deux directions au sein d'une empreinte bidimensionnelle de déplacement dudit boîtier lorsque l'ensemble est fixé sur la surface de corps d'animal.

4. Ensemble formant transducteur à ultrasons pouvant être manipulé à distance selon la revendication 1 ou 2, dans lequel ledit moteur permettant une rotation est en outre **caractérisé par** un engrenage couplé entre ledit moteur de rotation et ledit réseau de transducteurs linéaire pour faire tourner de manière incrémentielle ledit réseau de transducteurs linéaire selon un angle de rotation allant jusqu'à π rad (180 degrés) afin de collecter des données d'image d'une pluralité de plans d'image.

5. Ensemble formant transducteur à ultrasons pouvant être manipulé à distance selon la revendication 1 ou 2, permettant de coopérer avec un second ensemble similaire en vue d'une fixation au niveau d'une seconde position différente sur la surface externe de corps, **caractérisé en outre par** une capacité des premier et second ensembles coopérant à fournir une image stéréoscopique tridimensionnelle d'une région d'intérêt dudit corps d'animal sous la commande de dispositifs de commande respectifs et en réaction à des signaux de commande reçus grâce à des émetteurs-récepteurs sans fil respectifs.

6. Ensemble formant transducteur à ultrasons pouvant être manipulé à distance selon la revendication 1 ou 2, dans lequel ledit premier moteur permettant une rotation est en outre **caractérisé par** un engrenage associé permettant un déplacement rotationnel précis dudit réseau linéaire d'éléments transducteurs dudit diamètre en réaction à des signaux de commande sans fil à distance reçus au niveau dudit émetteur-récepteur sans fil.

7. Ensemble formant transducteur à ultrasons pouvant être manipulé à distance selon la revendication 1 ou 2, **caractérisé en outre par** :
une mémoire pour un stockage temporaire de données d'image capturées à partir d'une rotation dudit réseau de transducteurs linéaire, permettant une émission par l'intermédiaire dudit émetteur-récepteur sans fil.

8. Ensemble formant transducteur à ultrasons pouvant être manipulé à distance selon la revendication 1 ou 2, **caractérisé en outre par** :
une antenne, couplée audit émetteur-récepteur sans fil, permettant de recevoir des signaux radio sans fil à partir dudit poste de travail à distance et permettant d'émettre des signaux radio sans fil vers ledit poste de travail à distance.

9. Ensemble formant transducteur à ultrasons pouvant être manipulé à distance selon la revendication 1 ou 2, **caractérisé en outre par** :
une alarme déclenchable permettant de signaler à un utilisateur un état prédéterminé en réaction à une réception d'un signal d'activation à partir d'un site à distance par l'intermédiaire dudit émetteur-récepteur sans fil.

10. Ensemble formant transducteur à ultrasons pouvant être manipulé à distance selon la revendication 2, **caractérisé en outre par** :
un dispositif de commande permettant de commander ledit moteur de rotation en fonction d'entrées de commande à distance ;
une alimentation sur batterie permettant d'alimenter en énergie des éléments de l'ensemble formant transducteur nécessitant de l'énergie et ;
une mémoire permettant un stockage temporaire de données d'image capturées à partir d'une rotation dudit réseau de transducteurs linéaire.

11. Ensemble formant transducteur à ultrasons pouvant être manipulé à distance selon la revendication 1 ou 2, **caractérisé en outre par** :
ledit émetteur-récepteur sans fil signalant des données de position réelle d'un parmi ledit réseau de transducteurs linéaire à un poste de travail à distance, comprenant une rotation, une déformation et des emplacements x et y,
dans lequel lesdites données de position réelle comprennent des valeurs incluant l'allumage/l'extinction du réseau de transducteurs linéaire, la focalisation, la profondeur, l'angle de rotation et l'angle de déformation.

12. Ensemble formant transducteur à ultrasons pouvant être manipulé à distance selon la revendication 7, **caractérisé en outre par** :
une sortie dudit réseau de transducteurs linéaire fournie vers un convertisseur analogique-numérique permettant un échantillonnage préalablement au stockage dans ladite mémoire.

13. Ensemble formant transducteur à ultrasons pouvant être manipulé à distance selon la revendication 12, **caractérisé en outre en ce que**
ladite sortie dudit réseau de transducteurs linéaire est compressée au niveau d'un compresseur de données réagissant audit convertisseur analogique-numérique préalablement à ladite émission par l'intermédiaire dudit émetteur-récepteur sans fil.

14. Ensemble formant transducteur à ultrasons pouvant être manipulé à distance selon la revendication 1 ou 2, **caractérisé en outre par** :
une mémoire amovible couplée audit dispositif de commande, permettant de stocker et de télécharger des données d'image vers un poste de travail.

15. Ensemble formant transducteur à ultrasons pouvant être manipulé à distance selon la revendication 1 ou 2, dans lequel ledit réseau de transducteurs linéaire est **caractérisé en outre par** :
un code d'identification unique permettant d'identifier de manière unique ledit réseau de transducteurs auprès d'un poste de travail à distance.

16. Ensemble formant transducteur à ultrasons pouvant être manipulé à distance selon la revendication 7, **caractérisé en outre en ce que**
des données d'image émises sans fil à partir de ladite mémoire de dispositif de commande par l'intermédiaire dudit émetteur-récepteur sans fil vers un poste de travail à distance sont ensuite réémises vers et reçues au niveau dudit émetteur-récepteur sans fil dudit ensemble en vue d'une vérification avec lesdites données d'image émises sans fil stockées dans ladite mémoire de dispositif de commande.

17. Ensemble formant transducteur à ultrasons pouvant être manipulé à distance selon la revendication 1 ou 2, **caractérisé en outre par**
une entrée permettant de commander le réseau de transducteurs linéaire afin de fournir des ondes ultrasonores thérapeutiques vers une région spécifiée du corps d'animal, ladite entrée étant en outre capable de modifier un angle parmi un angle de rotation et un angle de déformation d'une propagation par ledit réseau de transducteurs linéaire.

18. Ensemble formant transducteur à ultrasons pouvant être manipulé à distance selon la revendication 1 ou 2, **caractérisé par** le premier réseau de transducteurs linéaire fonctionnant dans une première plage de fréquence pour un traitement thérapeutique et dans une seconde plage de fréquence supérieure à ladite première plage de fréquence pour une imagerie.

19. Ensemble formant transducteur à ultrasons pouvant être manipulé à distance selon la revendication 1 ou 2, dans lequel ledit ensemble est en outre **caractérisé par** :
une structure de tige sous la forme d'un rectangle comprenant deux paires de tiges parallèles formant le rectangle,
chaque paire parallèle de tiges guidant un autre élément de tige perpendiculaire dans une direction parmi des directions d'axe x et d'axe y, le réseau de transducteurs permettant un raccordement à la jonction des deux éléments de tiges perpendiculaires, les deux éléments de tiges perpendiculaires permettant un déplacement dudit réseau de transducteurs tel que déterminé par le troisième moteur d'axe x et le quatrième moteur d'axe y et en réaction à des données de commande reçues au niveau dudit émetteur-récepteur sans fil.

20. Ensemble formant transducteur à ultrasons pouvant être manipulé à distance selon la revendication 19, dans lequel lesdites tiges sont flexibles et peuvent être adaptées en combinaison pour se conformer à une aire de surface d'animal non plane.

21. Ensemble formant transducteur à ultrasons pouvant être manipulé à distance selon la revendication 1 ou 2, **caractérisé en outre par** :
une entrée de commande à distance filaire vers le dispositif de commande de l'ensemble, permettant une utilisation par un utilisateur humain de l'ensemble lors de la commande du fonctionnement de l'ensemble,
l'entrée de commande à distance filaire étant adaptée pour commander une sortie d'ondes sonores de manière thérapeutique vers une région d'intérêt en entrant un angle de déformation, un angle de rotation et une profondeur d'émission par l'intermédiaire de ladite commande à distance filaire.

22. Ensemble formant transducteur à ultrasons pouvant être manipulé à distance selon la revendication 1 ou 2, **caractérisé en outre par** une coopération avec un cathéter guidé par image permettant une imagerie d'une région d'intérêt dudit corps d'animal, le cathéter guidé par image permettant une utilisation dans des procédures chirurgicales avec effraction minimale en vue d'un objectif parmi des objectifs thérapeutiques, diagnostiques et interventionnels au niveau de ladite région d'intérêt.
